# FASCICULE DE BREVET EUROPEEN

(11) **EP 2 021 485 B1**
(45) Date de publication et mention de la délivrance du brevet: **01.12.2010**
(21) Numéro de dépôt: 07731461.5
(22) Date de dépôt: 15.05.2007
(51) Int. Cl.: C12N 15/90, C12N 15/10, C12N 9/22, C12N 15/82, A61K 48/00

(54) **PSEUDOTRANSPOSONS ET SYSTEMES DE TRANSPOSITION RECOMBINANTS HYPERACTIFS, DERIVES DU TRANSPOSON MOS-1**
PSEUDOTRANSPOSONS UND REKOMBINANTE HYPERAKTIVE TRANSPOSITIONSSYSTEME, ABGELEITET VOM MOS-1-TRANSPOSON
PSEUDOTRANSPOSONS AND RECOMBINANT HYPERACTIVE TRANSPOSITION SYSTEMS, DERIVED FROM THE MOS-1 TRANSPOSON

(30) Priorité: 15.05.2006 FR 0604285
(43) Date de publication de la demande: 11.02.2009
(73) Titulaire: CENTRE NATIONAL DE LA RECHERCHE SCIENTIFIQUE (CNRS), 75016 Paris (FR); Universite Francois Rabelais De Tours, 37000 Tours (FR)
(72) Inventeur: BIGOT, Sylvie, Marie-Louise, 37270 Montlouis sur Loire (FR); BIGOT, Yves, Philippe, Marcel, 37550 Saint Avertin (FR); BONNIN-ROULEUX, Florence, 37300 Joue les Tours (FR); GERMON, Stéphanie, Odile, Rébecca, 37380 Nouzilly (FR); JEGOT, Gwenhael, 29840 Landunvez (FR)
(74) Mandataire: Warcoin, Jacques
(86) Numéro de dépôt international: PCT/FR2007/000823
(87) Numéro de publication internationale: WO 2007/132096

(56) Documents cités:
- WO-A1-2004/078981
- US-B1- 6 368 830
- PLEDGER ET AL: "Mutant Mos1 mariner transposons are hyperactive in Aedes aegypti" INSECT BIOCHEMISTRY AND MOLECULAR BIOLOGY, ELSEVIER SCIENCE LTD, GB, vol. 35, no. 10, octobre 2005 (2005-10), pages 1199-1207, XP005019657 ISSN: 0965-1748
- ZHANG L ET AL: "DNA-binding activity and subunit interaction of the mariner transposase" NUCLEIC ACIDS RESEARCH, OXFORD UNIVERSITY PRESS, SURREY, GB, vol. 29, no. 17, 1 septembre 2001 (2001-09-01), pages 3566-3575, XP002256851 ISSN: 0305-1048
- AUGE-GOUILLOU C ET AL: "The wild-type conformation of the Mos-1 inverted terminal repeats is suboptimal for transposition in bacteria" MGG - MOLECULAR GENETICS AND GENOMICS, SPRINGER, BERLIN, DE, vol. 265, no. 1, mars 2001 (2001-03), pages 51-57, XP002255722 ISSN: 1617-4615 cité dans la demande
- PLEDGER D W ET AL: "Analyses of cis -acting elements that affect the transposition of Mos1 mariner transposons in vivo" MGG MOLECULAR GENETICS AND GENOMICS, vol. 272, no. 1, août 2004 (2004-08), pages 67-75, XP002426357 ISSN: 1617-4615
- TOSI LUIZ R O ET AL: "cis and trans factors affecting Mos1 mariner evolution and transposition in vitro, and its potential for functional genomics" NUCLEIC ACIDS RESEARCH, vol. 28, no. 3, 1 février 2000 (2000-02-01), pages 784-790, XP002426358 ISSN: 0305-1048 cité dans la demande
- DATABASE PDB E.B.I. Hinxton U.K.; 1 décembre 2005 (2005-12-01), RICHARDSON J.M. ET AL: "CRYSTAL STRUCTURE OF THE CATALYTIC DOMAIN OF MOS1 MARINER TRANSPOSASE" XP002426362 Database accession no. 2f7t -& RICHARDSON JULIA M ET AL: "Mechanism of Mos1 transposition: insights from structural analysis" EMBO (EUROPEAN MOLECULAR BIOLOGY ORGANIZATION) JOURNAL, vol. 25, no. 6, mars 2006 (2006-03), pages 1324-1334, XP002426359 ISSN: 0261-4189

## Description

La présente invention se rapporte au domaine de la biologie moléculaire relative aux éléments transposables. L'invention concerne plus particulièrement l'amélioration des propriétés des systèmes de transposition MOS-1 aux fins de leur utilisation en biotechnologies.

La présente invention A pour objet un Pseudo-transposon *Mos-1* hyperactif, dans lequel :
a) au moins l'une des deux répétitions terminales non traduites (UTR) et/ou au moins l'une des deux répétitions terminales inversées (ITR) est(sont) génétiquement modifiée(s) ; et
b) une séquence nucléotidique exogène d'intérêt remplace la séquence nucléotidique codant la transposase Mos-1 d'origine,
   **caractérisé en ce qu**'il est choisi parmi les pseudo-transposons suivants :
   α) ITR3'-UTR3'-séquence nucléotidique exogène d'intérêt-UTR3'-ITR3' (pseudo-transposon 33seq33),
   β) ITR3'-séquence nucléotidique exogène d'intérêt-UTR3'-ITR3' (pseudo-transposon 3seq33),
   γ) ITR3'-UTR5'-séquence nucléotidique exogène d'intérêt-UTR3'-ITR5' (pseudo-transposon 35seq35),
   δ) les pseudo-transposons comprenant au moins une ITR40 de séquence SEQ ID n°39, et
   ε) les pseudo-transposons comprenant au moins une ITR46 de séquence SEQ ID n°38, et dans lequel ITR3', UTR3', UTR5' et ITR5' désignent respectivement la séquence de la répétition terminale inversée 3' du transposon Mos-1, la séquence de la répétition terminale non-traduite 3' du transposon Mos-1, la séquence de la répétition terminale non-traduite 5' du transposon Mos-1 et la séquence de la répétition terminale inversée 5' du transposon Mos-1.

La présente invention a en outre pour objet un

Système de transposition recombinant hyperactif dérivé du transposon *Mos-1*, comprenant au moins les deux partenaires suivants :
a) un pseudo-transposon *Mos-1* hyperactif selon la revendication 1 ; et
b) une transposase Mos-1 fournie en *trans* dudit pseudo-transposon, dans lequel la fréquence de transposition de ladite séquence nucléotidique exogène d'intérêt est améliorée d'un facteur au moins égal à 5, de préférence au moins égal à 10, par rapport à la fréquence de transposition de la-dite séquence nucléotidique exogène d'intérêt portée par un pseudo-transposon Mos-1 bordé de deux ITR3' (3seq3) en présence de la transposase Mos-1 fournie en *trans*.

Outre de tels systèmes, l'invention fournit des cellules hôtes, des kits et des utilisations tels ue spécifiés dans les revendications.

La présente invention concerne également l'utilisation d'un ou plusieurs des moyens ci-dessus pour réaliser des transpositions de séquences, et plus particulièrement des transferts de gènes, efficaces dans une cellule eucaryote, à l'exception d'une cellule dans un organisme humain ou animal.

Les éléments transposables (TE) ou éléments génétiques mobiles (EGM) sont des fragments d'ADN de petite taille, capables de se déplacer d'un site chromosomique à un autre (Renault *et al*., 1997). Ces fragments d'ADN sont caractérisés par des séquences répétées inversées (ITR) situées en positions 5' et 3' terminales. Une enzyme codée par les TE eux-mêmes, la transposase, catalyse le processus de transposition de ces derniers.

Des TEs ont été identifiés tant chez les procaryotes que chez les eucaryotes (voir un ouvrage de référence dans ce domaine : Craig *et al*., 2002).

Les TEs sont répartis en deux classes d'après leur mécanisme de transposition. D'une part, les éléments de classe I, ou rétrotransposons, transposent *via* la transcription inverse d'un intermédiaire ARN. D'autre part, les éléments de classe II transposent directement d'un site chromosomique à un autre, *via* un intermédiaire ADN, selon un mécanisme de type « couper-coller ».

Chez les procaryotes, un grand nombre de TEs ont été répertoriés à ce jour. L'on peut citer, par exemple, des séquences d'insertion telles que IS*1*, et des transposons, tel Tn*5*.

Chez les eucaryotes, les éléments de classe II comprennent dix tamilles : P, PiggyBac, hAT, hélitron, Harbinger, En/Spm, Mutator, Transib, Pogo et Tc1-mariner.

Les éléments génétiques mobiles *mariner* (ou MLE pour « *mariner*-like éléments ») constituent un grand groupe de TEs de classe II, appartenant à la superfamille Tc1-mariner (Plasterk *et al.,* 1999).

La capacité des transposases de TE à mobiliser des fragments d'ADN plus ou moins longs, homologues ou hétérologues, comprenant des séquences d'intérêt, pour les insérer dans des acides nucléiques cibles, en particulier dans le chromosome d'un hôte, a été et est encore largement mise à profit dans le domaine des biotechnologies, notamment dans le domaine du génie génétique.

Parmi les TEs, les MLEs présentent des propriétés particulièrement avantageuses pour une utilisation en biotechnologie, notamment en ingénierie génétique et génomique fonctionnelle. L'on peut par exemple citer de manière non limitative les propriétés suivantes :
(i) Les MLEs sont des transposons de petite taille, faciles à manipuler.
(ii) Le mécanisme de transposition des MLEs est simple. En effet, la transposase est capable de catalyser, à elle seule, toutes les étapes de la transposition des MLEs. Elle est d'ailleurs nécessaire et suffisante pour assurer la mobilité des MLEs en l'absence de facteurs de l'hôte (Lampe *et al*., 1996).
(iii) Les MLEs se caractérisent par une très large ubiquité chez les procaryotes et les eucaryotes. Le premier MLE, *Dmmar1,* également appelé *Mos-1,* a été découvert chez *Drosophila mauritiana* par Jacobson et Harti (1985). Par la suite, de nombreux éléments apparentés ont été identifiés dans des génomes appartenant notamment à des bactéries, protozoaires, champignons, plantes, invertébrés, vertébrés à sang froid et mammifères.
(iv) L'activité transpositionnelle des MLEs est hautement spécifique, et n'induit pas de mécanismes de « résistance » du génome de l'hôte, tels que des phénomènes d'interférences par méthylation [MIP ; Jeong *et al.* (2002) ; Martienssen et Colot (2001)] ou via des ARN [RNAi; Ketting *et al.* (1999) ; Tabara *et al.* (1999)]. Les événements de transposition peuvent être contrôlés par divers facteurs, tels la température, la présence de certains cations divalents et le pH.

En termes de structure, l'élément *Mos-1* de *mariner* est un élément compact de 1286 pb contenant un seul cadre ouvert de lecture (ORF) qui code pour une transposase de 354 acides aminés. La transposase est constituée d'un domaine N-terminal impliqué dans la liaison à l'ADN, la dimérisation et la tétramérisation et d'un domaine C-terminal contenant le site actif où un motif DDE(D) coordine l'ion métallique catalytique. Le cadre de lecture est flanqué de deux séquences terminales inversées (ITR) de 28±2 pb. Les deux régions localisées entre les ITR et l'ORF ne sont pas traduites et sont appelées UTR (pour « Untranslated Terminal Region »). Les deux ITR de *Mos-1* diffèrent en séquence par quatre nucléotides, ce qui laisse penser que la configuration naturelle de cet élément n'est pas optimale pour la transposition. Ceci a d'ailleurs été vérifié par des expériences montrant qu'un pseudo-transposon bordé par deux ITR 3' de *Mos-1* transpose 10 000 fois mieux *in vivo* en bactérie que celui bordé par les ITR 5' et 3' en configuration naturelle (Augé-Gouillou et al., 2001b).

Les applications potentielles des MLEs en biotechnologie, notamment comme outils non-viraux de recombinaison génétique, sont considérables.

Typiquement, pour des applications de mutagenèse insertionnelle *in vitro,* le gène du transposon qui code pour la transposase est remplacé par un ADN « étiquette ». La transposase est fournie en *trans* sous forme protéique. Pour des applications de transfert de gènes *in vivo* ou *in vitro,* le gène codant la transposase est remplacé par l'ADN exogène à transférer (on obtient ainsi un « pseudo-transposon »). La transposase est alors fournie en *trans* via un plasmide d'expression, un ARN messager ou la protéine elle-même. Toutefois, le transfert d'un ADN exogène à l'aide des moyens actuels, c'est-à-dire en utilisant un pseudo-transposon *mariner,* n'est pas sans poser des difficultés, notamment du fait d'une efficacité et d'une spécificité d'intégration du transgène très limitées.

Le document par Pledger D et Coates C (2005) dans Insect Biochemistry and Molecular Biology vol.35 pp. 1199-1207 décrit l'augmentation de l'activité de transposition d'un pseudo-transposon Mos-1 lorsqu'une transposase Mos-1 mutée hyperactive est utilisée en combinaison avec un pseudo-transposon hyperactif 3T3 (selon la terminologie de la présente demande).

Le document par Augé-Gouillou C et al (2001b) dans Molecular Genetics and Genomics vol.265 pp.51-57 décrit que l'affinité de la transposase Mos-1 pour l'ITR3' est supérieure à celle pour l'ITR5', et que l'activité de transposition est plus élevée sur un pseudo-transposon comprenant deux ITR3' (3T3) que sur un pseudo-transposon comprenant une ITR5' and une ITR3'.

Le document par Pledger D.et al dans Molecular Genetics and Genomics vol.272 pp.67-75 décrit l'augmentation d'activité de transposition dans E. coli lorsque les deux ITRs sont de type 3', et décrit une augmentation supplémentaire lorsque la séquence interne à Mos-1 sauf 10 paires de base est enlevée.

Il est pourtant essentiel de disposer, dans chacune de ces applications, de systèmes de transposition efficaces.

Or, l'efficacité de transposition des MLE naturels, comparée à celle d'autres TE, reste faible. En particulier, les MLE semblent moins actifs chez les eucaryotes que les autres EGM de classe II. En définitive, l'intérêt pratique des MLE naturels est demeuré jusqu'à présent limité, puisqu'il est important, pour l'industriel ou le chercheur, de disposer d'outils de transposition efficaces, de manière à réduire le nombre de manipulations, le coût et le temps nécessaires pour réaliser les transpositions recherchées. Faute d'efficacité suffisante des systèmes de transposition disponibles, l'industriel ou le chercheur est aujourd'hui enclin à privilégier, dans la mesure du possible, l'utilisation de systèmes viraux de transfert de gènes - et ce, malgré les inconvénients qu'ils présentent - au détriment des systèmes de transposition recombinants construits à partir des transposons MLE.

Il existe donc à ce jour un besoin pour un système qui (i) permette de transférer efficacement des gènes ; (ii) présente une bonne innocuité en terme d'immunogénicité pour l'hôte ; (iii) garantisse la sécurité de l'hôte et de l'environnement (absence de contaminations, notamment absence d'émergence de virus recombinants) ; (iv) soit facile à produire.

La présente invention permet précisément de répondre à ce besoin en fournissant un système de transposition recombinant qui met à profit les avantages de l'élément *Mos-1* (ubiquité, activité transpositionnelle, simplicité de production et de mise en oeuvre, etc.), tout en remédiant aux inconvénients liés à la faible efficacité de transposition de cet élément à l'état sauvage.

Ainsi, afin de répondre de manière satisfaisante au besoin existant, les Inventeurs se sont intéressés à l'élément MLE *Mos-1* de *Drosophile mauritiana* qui est le membre le plus caractérisé de cette famille et le seul qui soit naturellement actif. En outre, l'élément *Mos-1* présente l'avantage considérable d'être actif à la fois en cellule eucaryote et en bactérie, ce qui rend son intérêt évident dans le cadre de la mise au point d'un système de transposition ubiquiste efficace.

Comme il ressort des différents aspects de la présente invention décrits dans le détail ci-après, les Inventeurs proposent plusieurs outils qui améliorent le système de transposition *Mos-1* naturel. Ces outils, qui peuvent être utilisés seuls ou en combinaison en fonction des applications envisagées, comprennent :
des pseudo-transposons *Mos-1* recombinants hyperactifs.

On définit ici un « pseudo-transposon » comme étant un transposon dans lequel le gène codant pour la transposase a été remplacé par une séquence nucléotidique exogène. Un pseudo-transposon possède donc des extrémités ITR et UTR mais est dépourvu de l'activité transposase. Il a par conséquent perdu la capacité à transposer, sauf à être associé à une transposase extérieure.

Ainsi, les Inventeurs se sont intéressés à des systèmes de transposition recombinants hyperactifs dérivés du transposon *Mos-1*, comprenant au moins les deux partenaires suivants :
a) un pseudo-transposon *Mos-1* dans lequel une séquence nucléotidique exogène d'intérêt remplace la séquence nucléotidique codant la transposase Mos-1 d'origine ; et
b) une transposase Mos-1 fournie en *trans* dudit pseudo-transposon, l'un au moins desdits partenaires étant génétiquement modifié de manière appropriée pour améliorer la fréquence de transposition de ladite séquence nucléotidique exogène d'intérêt d'un facteur au moins égal à 5, de préférence au moins égal à 10.

Dans les systèmes de transposition recombinants proposés, les deux partenaires, à savoir le pseudo-transposon et la transposase, peuvent être génétiquement modifiés. Ainsi, l'un ou l'autre des partenaires ou les deux peu(ven)t être génétiquement modifié(s) et hyperactif(s).

Une « séquence nucléotidique » ou un « acide nucléique » selon l'invention est conforme à l'acception usuelle dans le domaine de la biologie. Ces deux expressions couvrent indifféremment les ADN et les ARN, les premiers prouvant être par exemple génomiques, plasmidiques, recombinants, complémentaires (ADNc), et les seconds, messagers (ARNm), ribosomaux (ARNr), de transfert (ARNt). De manière préférée, les séquences nucléotidiques et acides nucléiques de l'invention sont des ADN.

Les termes et expressions « activité », « fonction », « activité biologique » et « fonction biologique » sont équivalents et répondent à l'acception usuelle dans le domaine technique de l'invention. Dans le cadre de l'invention, l'activité en cause est l'activité de transposition.

L'« hyperactivité » correspond ici à une activité de transposition supérieure à celle observée en utilisant un système de transposition *Mos-1* naturel comprenant :
a) un pseudo-transposon *Mos-1* dans lequel une séquence nucléotidique exogène d'intérêt remplace la séquence nucléotidique codant la transposase Mos-1 d'origine ; et
b) la transposase Mos-1 sauvage, fournie en *trans* dudit pseudo-transposon.

De surcroît, l'« hyperactivité » au sens de l'invention désigne une activité de transposition supérieure à celle observée en utilisant un système de transposition *Mos-1* recombinant comprenant :
a) un pseudo-transposon *Mos-1* dans lequel
   (i) une séquence nucléotidique exogène d'intérêt remplace la séquence nucléotidique codant la transposase Mos-1 d'origine, et
   (ii) la répétition terminale inversée sauvage située en 5' (ITR 5') a été mutée de sorte qu'elle est une copie parfaite de la répétition terminale inversée sauvage située en 3' (ITR 3') ; et
b) la transposase Mos-1 sauvage, fournie en *trans* dudit pseudo-transposon.

Ce pseudo-transposon est donc bordé de 2 ITR 3' (« pseudo-transposon 2 ITR 3' » ou « pseudo-transposon 3T3 »). La présence de 2 ITR 3' ayant déjà été décrite comme permettant d'améliorer l'efficacité de transposition par rapport au transposon *Mos-1* naturel (Augé-Gouillou et al., 2001b), le système recombinant ci-dessus est donc utilisé ici comme référence pour déterminer si un système de transposition recombinant est « hyperactif » au sens de la présente invention. Le système recombinant de référence ci-dessus décrit est désigné dans la suite comme étant le « système (de transposition) de référence » ou « système (de transposition) 3T3 ».

L'« efficacité de transposition » se détermine d'après la fréquence de transposition. L'efficacité de la transposition est donc améliorée si la fréquence de la transposition est augmentée. On parlera dans ce qui suit indifféremment d'« amélioration de l'activité (de transposition) », d'« amélioration de la transposition » ou encore d'« amélioration de l'efficacité (de transposition) », toutes ces expressions renvoyant à l'« amélioration de la fréquence de transposition », c'est-à-dire à son augmentation.

Afin de quantifier l'« hyperactivité », on utilise ici un « facteur » ou « facteur d'hyperactivité » qui est égal au rapport des fréquences de transposition conformément à la formule suivante :
Facteur d'hyperactivité (F) = (fréquence de transposition observée avec un système de transposition recombinant donné) / (fréquence de transposition observée avec le système de transposition de référence 3T3 défini supra).

En d'autres termes, on compare la fréquence de transposition d'une séquence nucléotidique exogène portée par un pseudo-transposon *Mos-1* en présence de la transposase Mos-1 fournie en *trans,* à la fréquence de transposition de cette même séquence nucléotidique exogène lorsqu'elle est portée par le pseudo-transposon *Mos-1* de référence en présence de la transposase Mos-1 fournie en trans (système 3T3). Cette méthode d'évaluation de l'activité de transposition est une pratique des plus courantes dans le domaine.

Les systèmes de transposition recombinants intéressants dans le cadre de la présente invention permettent donc d'améliorer la transposition d'un facteur au moins égal à 5. De préférence, le facteur d'hyperactivité est au moins égal à 10 et, mieux, il est au moins égal à 15. De manière encore préférée, il est au moins égal à 20, 25, 30, 35, 40, 45, 50, 55, 60, et plus.

Comme il ressort des exemples ci-après, la ou les modifications génétiques du pseudo-transposon et/ou de la transposase est(sont) précisément sélectionnée(s) pour sa(leur) capacité à entraîner une hyperactivité de transposition. Les mutations et les combinaisons de mutations qui rendent la transposase et/ou le pseudo-transposon et/ou le système de transposition hyperactifs ne sont ni aléatoires, ni prévisibles. Aux fins de la sélection des mutations appropriées, les inventeurs ont utilisé un test de transposition bien connu dans le domaine. Ce test a déjà été décrit dans la littérature (notamment dans Augé-Gouillou et al., 2001 b) et fait partie des connaissances générales de l'homme du métier qui pourra néanmoins, s'il le souhaite, utiliser n'importe quel autre test adapté à sa disposition.

Un premier aspect de la présente invention concerne un Pseudo-transposon *Mos-1* hyperactif, dans lequel :
a) au moins l'une des deux répétitions terminales non traduites (UTR) et/ou au moins l'une des deux répétitions terminales inversées (ITR) est(sont) génétiquement modfiée(s) ; et
b) une séquence nucléotidique exogène d'intérêt remplace la séquence nucléotidique codant la transposase Mos-1 d'origine,
   **caractérisé en ce qu**'il est choisi parmi les pseudo-transposons suivants :
   α) ITR3'-UTR3'-séquence nucléotidique exogène d'intérêt-UTR3'-ITR3' (pseudo-transposon 33seq33),
   β) ITR3'-séquence nucléotidique exogène d'intérét-UTR3'-ITR3' (pseudo-transposon 3seq33),
   γ) ITR3'-UTR5'-séquence nucléotidique exogène d'intérêt-UTR3'-ITR5' (pseudo-transposon 35seq35),
   δ) les pseudo-transposons comprenant au moins une ITR40 de séquence SEQ ID n°39, et
   ε) les pseudo-transposons comprenant au moins une ITR46 de séquence SEQ ID n°38, et dans lequel ITR3', UTR3', UTR5' et ITR5' désignent respectivement la séquence de la répétition terminale inversée 3' du transposon Mos-1, la séquence de la répétition terminale non-traduite 3' du transposon Mos-1, la séquence de la répétition terminale non-traduite 5' du transposon Mos-1 et la séquence de la répétition terminale inversée 5' du transposon Mos-1.

un second aspect de l'invention concerne un Système de transposition recombinant hyperactif dérivé du transposon *Mos-1*, comprenant au moins les deux partenaires suivants :
a) un pseudo-transposon *Mos-1* hyperactif selon la revendication 1 ; et
b) une transposase Mos-1 fournie en *trans* dudit pseudo-transposon, dans lequel la fréquence de transposition de ladite séquence nucléotidique exogène d'intérêt est améliorée d'un facteur au moins égal à 5, de préférence au moins égal à 10, par rapport à la fréquence de transposition de la-dite séquence nucléotidique exogène d'intérêt portée par un pseudo-transposon Mos-1 bordé de deux ITR3' (3seq3) en présence de la transposase Mos-1 fournie en *trans*.

D'autres aspects de l'invention sont détaillés dans les revendications.

Dans le cadre de la présente invention, les pseudo-transposons sont désignés de la manière suivante : ITR - UTR - séquence nucléotidique exogène d'intérêt - UTR - ITR. Dans le cas de l'exemple particulier ITR 3' - UTR 3' - séquence nucléotidique exogène d'intérêt - UTR 3' - ITR 3', cela donne en abrégé 33seq33 ou 33T33. La désignation 33T33 pourra éventuellement faire plus particulièrement référence au pseudo-transposon dans lequel la séquence nucléotidique exogène d'intérêt est le gène de résistance à la tétracycline (voir notamment les exemples ci-dessous). Bien entendu, ceci ressortira clairement du contexte.

Comme indiqué plus haut, la transposase Mos-1 fournie en trans dans ce système pourra être une transposase Mos-1 mutante et, en particulier, une transposase Mos-1 mutante hyperactive. Par exemple, une transposase Mos-1 mutante hyperactive appropriée comprendra au moins une mutation au niveau d'au moins un résidu choisi parmi les résidus suivants de la séquence SEQ ID N°2 : F53, Q91, E137, T216 et Y237.

Ainsi, des systèmes de transposition préférés au sens de la présente invention comprennent au moins les deux partenaires suivants :
a) un pseudo-transposon *Mos-1* hyperactif comprenant au moins une ITR40 de séquence SEQ ID n°39 et une transposase Mos-1 mutante hyperactive comprenant les mutations T216A et Y237C ;
b) un pseudo-transposon *Mos-1* hyperactif comprenant au moins une ITR46 de séquence SEQ ID n°38 et une transposase Mos-1 mutante hyperactive comprenant les mutations T216A et Y237C, ou E137K et T216A, ou F53Y et T216A et Y237C ;
c) un pseudo-transposon *Mos-1* hyperactif 3seq33 et une transposase Mos-1 mutante hyperactive comprenant les mutations T216A et Y237C, ou E137K et T216A, ou encore F53Y et Q91R, ou bien encore F53Y et Q91R et E137K et T216A.
Aussi décrit est un système de transposition recombinant hyperactif comprenant au moins les deux partenaires suivants :
a) un pseudo-transposon *Mos-1* dans lequel une séquence nucléotidique exogène d'intérêt remplace la séquence nucléotidique codant la transposase Mos-1 d'origine ; et
b) une transposase Mos-1 hyperactive fournie en *trans* dudit pseudo-transposon et comprenant au moins :
   - une mutation au niveau d'au moins un résidu choisi parmi les résidus suivants de la séquence SEQ ID N°2 : F53, Q91 et Y237, et/ou
   - la mutation T216A,
la fréquence de transposition de la séquence nucléotidique exogène d'intérêt de ce système étant améliorée d'un facteur au moins égal à 5, de préférence au moins égal à 10.

De préférence, la transposase Mos-1 hyperactive comprend au moins une mutation choisie parmi les mutations F53Y, Q91R, T216A, Y237C, et leurs combinaisons. Elle peut en outre comprendre une mutation au niveau du résidu E137, notamment la mutation E137K, mais à l'exclusion toutefois de la combinaison de mutations Q91R+E137K+T216A ou F53Y+E137K+T216A.

Avantageusement, dans un tel système, au moins l'une des deux répétitions terminales non traduites (UTR) et/ou au moins l'une des deux répétitions terminales inversées (ITR) du pseudo-transposon *Mos-1* pourra(pourront) être génétiquement modifiée(s).

Dans les systèmes de transposition conformes à la présente invention, la transposase Mos-1, sauvage ou génétiquement modifiée, est fournie en *trans* du pseudo-transposon. Elle peut ainsi être codée par une séquence nucléotidique placée sur un vecteur, sous le contrôle d'éléments de régulation de l'expression. Avantageusement, l'expression de la transposase sera alors inductible. Pour cela, des promoteurs conventionnels pourront être utilisés par l'homme du métier. Par exemple, il pourra utiliser le promoteur bien connu *pLac* inductible à l'IPTG. Alternativement, la transposase peut être ajoutée dans le système de transposition sous la forme d'une protéine ou d'une fraction protéique fonctionnelle purifiée. Ou bien, elle peut être apportée sous la forme d'un ARN messager. En ce cas, l'expression de la transposase sera limitée dans le temps (à quelques heures) du fait de la labilité des ARN messagers.

De manière particulièrement intéressante, la séquence nucléotidique exogène d'intérêt portée par le pseudo-transposon *Mos-1* est un gène fonctionnel. Au sens de l'invention, un gène est dit « fonctionnel » si la séquence nucléotidique correspondante comprend au moins le cadre ouvert de lecture (ORF), c'est-à-dire la séquence codante, apte à donner lieu à une séquence en acides aminés présentant l'activité du produit du gène sauvage. De préférence, le gène fonctionnel est le gène sauvage. Il peut néanmoins s'agir d'un gène comprenant une ou plusieurs mutations dès lors que le produit du gène muté demeure actif (et ce, même si l'activité du produit du gène muté est plus faible que celle du produit du gène natif). Ainsi, dans cette définition de « gène fonctionnel », on englobe également les séquences codantes dépourvues de leur promoteur. A titre d'exemple, la séquence nucléotidique exogène d'intérêt peut être un gène de résistance à un antibiotique, dépourvu ou non de son promoteur (par exemple, le gène de résistance à la tétracycline), ou n'importe quel autre marqueur de sélection approprié.

Au sens de l'invention, une « mutation » est conforme à l'acception usuelle en biotechnologie. Ainsi, une mutation peut être une substitution, une addition ou une délétion d'une ou plusieurs bases dans une séquence nucléotidique, ou d'un ou plusieurs acides aminés dans une séquence protéique. Une « mutation » peut notamment désigner une substitution d'au moins une base d'un codon d'une séquence nucléotidique, ladite substitution entraînant par exemple, lors de la traduction de la séquence nucléotidique en cause, l'incorporation d'un acide aminé différent aux lieu et place de l'acide aminé natif, dans la séquence protéique résultante. En règle générale, on préférera que la ou les mutations n'entraîne(nt) pas de perte de la fonction biologique du produit muté. En revanche, une diminution d'activité pourra éventuellement être tolérée, sauf si l'élément muté (par exemple le pseudo-transposon et/ou la transposase) est « hyperactif », auquel cas son activité devra être supérieure à celle de l'élément sauvage correspondant.

On considère ainsi qu'une transposase est « hyperactive », ou qu'un pseudo-transposon est « hyperactif », si l'efficacité de transposition observée en la ou le mettant en oeuvre est accrue. L'augmentation d'activité de l'élément en question (pseudo-transposon ou transposase) est déterminée lors de sa mise en oeuvre dans un système de transposition recombinant conforme à l'invention. Le facteur d'hyperactivité peut ainsi être déterminé et, si le niveau d'activité transpositionnelle obtenu atteint le seuil fixé dans le cadre de l'invention, l'élément est dit « hyperactif ».

De manière générale, une « modification génétique » doit ici être entendue comme équivalant à une ou plusieurs mutations. Si une séquence codante est génétiquement modifiée, alors, typiquement, elle contient une ou plusieurs mutations. Ces mutations ne doivent cependant pas entraîner une perte de la fonction du produit codé. Au contraire, dans le cas, par exemple, d'une transposase Mos-1 génétiquement modifiée, on recherchera de préférence une augmentation de son activité (c'est-à-dire une transposase recombinante hyperactive). Si une séquence non codante est génétiquement modifiée (par exemple, une ITR ou une UTR), alors soit elle contient une ou plusieurs mutations, soit ce n'est pas la séquence individuelle en tant que telle qui est modifiée, mais le nombre de répétitions de cette séquence et/ou l'ordre dans l'enchaînement des séquences et/ou l'orientation de cette séquence par rapport aux autres, qui est(sont) modifié(e)(s) par rapport à la configuration normale (par exemple, par rapport au transposon *Mos-1* sauvage). A titre de d'exemples de modifications génétiques d'une séquence non codante telle qu'une ITR ou une UTR, on citera notamment la délétion de tout ou partie de la séquence, la permutation de la séquence avec d'autres séquences présentent dans l'environnement, etc. En somme, un agencement différent de séquences, qu'elles soient codantes ou non, rentre dans la définition des « modifications génétiques » selon l'invention.

Conformément à la description qui précède, on peut mettre en oeuvre, dans les systèmes de transposition recombinants visés par la présente invention, un Pseudo-transposon *Mos-1* hyperactif, dans lequel :
a) au moins l'une des deux répétitions terminales non traduites (UTR) et/ou au moins l'une des deux répétitions terminales inversées (ITR) est(sont) génétiquement modifiée(s) ; et
b) une séquence nucléotidique exogène d'intérêt remplace la séquence nucléotidique codant la transposase Mos-1 d'origine,
   **caractérisé en ce qu**'il est choisi parmi les pseudo-transposons suivants :
   α) ITR3'-UTR3'-séquence nucléotidique exogène d'intérêt-UTR3'-ITR3' (pseudo-transposon 33seq33),
   β) ITR3'-séquence nucléotidique exogène d'intérêt-UTR3'-ITR3' (pseudo-transposon 3seq33),
   γ) ITR3'-UTR5'-séquence nucléotidique exogène d'intérêt-UTR3'-ITR5' (pseudo-transposon 35seq35),
   δ) les pseudo-transposons comprenant au moins une ITR40 de séquence SEQ ID n°39, et
   ε) les pseudo-transposons comprenant au moins une ITR46 de séquence SEQ ID n°38, et dans lequel ITR3', UTR3', UTR5' et ITR5' désignent respectivement la séquence de la répétition terminale inversée 3' du transposon Mos-1, la séquence de la répétition terminale non-traduite 3' du transposon Mos-1, la séquence de la répétition terminale non-traduite 5' du transposon Mos-1 et la séquence de la répétition terminale inversée 5' du transposon Mos-1.

On exclut le pseudo-transposon 2 ITR 3' des pseudo-transposons *Mos-1* susceptibles d'être mis en oeuvre dans le cadre de l'invention. Comme indiqué plus haut, celui-ci est utilisé comme référence pour déterminer l'hyperactivité des systèmes de transposition selon l'invention.

Dans certains cas, au moins l'une des deux répétitions terminales non traduites (UTR) du pseudo-transposon *Mos-1* hyperactif est génétiquement modifiée. Alternativement ou additionnellement, au moins l'une des deux répétitions terminales inversées (ITR) du pseudo-transposon *Mos-1* hyperactif est génétiquement modifiée.

Conformément à ce qui précède et comme illustré dans les exemples ci-après, le ou les ITR génétiquement modifié(s) du pseudo-transposon *Mos-*1 recombinant hyperactif peu(ven)t être avantageusement choisi(s) parmi les séquences d'ITRSelex appelées ITR40 (SEQ ID N°39) et ITR46 (SEQ ID N°38).

Alternativement, le pseudo-transposon *Mos-1* recombinant hyperactif peut comprendre une combinaison d'ITR et UTR choisie notamment parmi les combinaisons suivantes: ITR 3' + UTR 5' / UTR 3' + ITR 5' (notée 35T35 ou 35seq35), ITR 3' + UTR 3' / UTR 3' + ITR 3' (notée 33T33 ou 33seq33). ITR 3' / UTR 3' + ITR 3' (notée 3T33 ou 3seq33).

Dans certains modes de réalisation, la transposase Mos-1 fournie en *trans* dans le système de transposition recombinant est une transposase hyperactive comprenant au moins une mutation au niveau d'au moins un résidu choisi parmi les résidus suivants de la séquence SEQ ID N°2 : F53, Q91, E137, T216 et Y237, à l'exclusion de la combinaison de mutations Q91R+E137K+T216A ou F53Y+E137K+T216A. Les résultats des expériences réalisées par les Inventeurs révèlent que l'on ne peut pas pratiquer n'importe quelle mutation ou combinaison de mutations sur la transposase Mos-1 pour obtenir une transposase recombinante hyperactive. En particulier, il apparaît (voir partie expérimentale ci-dessous) que les deux combinaisons de mutations ici exclues conduisent à une abolition de la transposition. Dans ces combinaisons exclues, les mutations peuvent donc être considérées comme antagonistes.

Avantageusement, la transposase Mos-1 hyperactive pourra comprendre au moins une mutation choisie parmi les mutations F53Y, Q91R, E137K, T216A, Y237C, et leurs combinaisons, à l'exclusion de la combinaison de mutations Q91R+E13TK+T216A ou F53Y+E137K+T216A.

De préférence, la transposase Mos-1 hyperactive pourra comprendre au moins une mutation au niveau du résidu T216. De préférence encore, elle pourra comprendre au moins la mutation T216A. Avantageusement, une telle transposase hyperactive pourra en outre comprendre au moins une mutation choisie parmi les mutations F53Y, Q91R, E137K, Y237C, et leurs combinaisons, à l'exclusion de la combinaison de mutations Q91R+E137K+T216A ou F53Y+E137K+T216A.

Comme il ressort de la partie expérimentale ci-dessous, des transposases Mos-1 hyperactives particulièrement intéressantes comprennent au moins l'une des combinaisons de mutations suivantes :
- T216A+Y237C ; F53Y+T216A+Y237C : facteur d'hyperactivité au moins égal à 20 ;
- F53Y+Q91 R+E137K+T216A+Y237C : facteur d'hyperactivité au moins égal à 30 ;
- F53Y+E137K+T216A+Y237C : facteur d'hyperactivité au moins égal à 45.

Selon un mode de réalisation alternatif ou additionnel, la transposase Mos-1 fournie en *trans* dans les systèmes de transposition recombinants de la présente invention est une transposase hyperactive comprenant au moins une mutation au niveau d'un résidu phosphorylable, ladite mutation étant suppressive d'un site de phosphorylation en cellule eucaryote (par exemple, cellule de plante, de vertébré ou d'invertébré). Là encore, la ou les mutations envisagées, suppressives d'un ou plusieurs site(s) de phosphorylation en cellule eucaryote, sont évidemment conservatives de la fonction enzymatique de la protéine. Des transposases mutantes hyperactives appropriées sont décrites dans la demande de brevet français N°03 00905 déposée le 28 janvier 2003. En particulier, ces transposases sont telles que le résidu phosphorylable muté est choisi parmi les résidus suivants de la séquence SEQ ID N°2 : T11, T24, S28, T42, T88, S99, S104, T135, S147. T154, S170, T181, S200. T216, T255 et S305. De préférence, la transposase hyperactive comprend au moins une mutation au niveau du résidu phosphorylable T88. Avantageusement, elle comprend en outre au moins une mutation au niveau des résidus phosphorylables T11, T24, S28, T42, S99, S104, T135, S147, T154, S170, T181, S200, T216, T255 et S305. En particulier, le ou lesdits résidus phosphorylables ainsi mutés sont substitués par un ou des résidus non phosphorylables en cellule eucaryote.

De manière générale dans le cadre de l'invention, la transposase Mos-1. recombinante hyperactive ou sauvage, peut avantageusement être produite de manière stable chez les procaryotes. Dans ce cas, un procédé approprié de production, par une cellule hôte procaryote, d'une transposase Mos-1 active (éventuellement, hyperactive) et stable comprend au moins les étapes suivantes :
a) le clonage de la séquence nucléotidique codant la transposase active dans un vecteur d'expression :
b) le clonage de la séquence nucléotidique codant la sous-unité catalytique active de la protéine kinase AMPc-dépendante (pKa) dans un vecteur d'expression ;
c) la transformation de ladite cellule hôte avec lesdits vecteurs d'expression ;
d) l'expression desdites séquences nucléotidiques par ladite cellule hôte ; et
e) l'obtention de la transposase active et stabilisée par phosphorylation par la pKa.
Alternativement, les clonages des étapes a) et b) sont réalisés dans un seul vecteur d'expression.
De manière intéressante, de tels procédés comprennent en outre une étape de purification de la transposase.
Pour plus de détails concernant ces procédés, l'homme du métier pourra se référer à la demande de brevet français FR 2893951 déposée le 30 novembre 2005.

Un premier aspect de la présente invention concerne un pseudo-transposon *Mos-1* hyperactif, dans lequel :
a) au moins l'une des deux répétitions terminales non traduites (UTR) et/ou au moins l'une des deux répétitions terminales inversées (ITR) est(sont) génétiquement modifiée(s) ; et
b) une séquence nucléotidique exogène d'intérêt remplace la séquence nucléotidique codant la transposase Mos-1 d'origine,
   ledit pseudo-transposon étant choisi parmi les pseudo-transposons suivants :
   α) ITR3'-UTR3'-séquence nucléotidique exogène d'intérêt-UTR3'-ITR3' (pseudo-transposon 33seq33).
   β) ITR3'-séquence nucléotidique exogène d'intérêt-UTR3'-ITR3' (pseudo-transposon 3seq33),
   γ) ITR3'-UTR5'-séquence nucléotidique exogène d'intérêt-UTR3'-ITR5' (pseudo-transposon 35seq35),
   δ) les pseudo-transposons comprenant au moins une ITR40 de séquence SEQ ID n°39, et
   ε) les pseudo-transposons comprenant au moins une ITR46 de séquence SEQ ID n°38.

D'autres aspects de l'invention sont détaillés dans les revendications

Une cellule hôte telle que définie dans les revendications est choisie parmi les cellules procaryotes (bactéries par exemple, notamment *Escherichia coli*) et les cellules eucaryotes (notamment, les cellules de plantes, de vertébrés et d'invertébrés).

Un kit tel que défini dans les revendications pourra en outre comprendre un ou plusieurs éléments choisis parmi, notamment, un tampon compatible avec la ou les transposases, un tampon "stop" pour arrêter les reactions de transposition, un ou plusieurs ADN contrôles (témoins de réaction), des oligonucléotides utiles pour le sequençage après réaction, des bactéries compétentes, une notice d'utilisation, etc.

Dans un mode de réalisation, l'un au moins de ces moyens est utilisé pour la transposition efficace *in vitro* ou *in vivo* (en particulier dans une cellule hôte de plante) ou *ex vivo* d'une séquence nucléotidique exogène d'intérêt dans une cellule eucaryote ou procaryote, à l'exception d'une cellule dans un organisme humain ou animal.

Selon un autre mode de réalisation, l'un au moins de ces moyens est utilisé pour la préparation d'un médicament destiné à permettre la transposition efficace *in vivo* d'une séquence nucléotidique exogène d'intérêt.

Alternativement, l'un au moins de ces moyens est utilisé pour la préparation d'un médicament résultant de la transposition *in vitro* ou *ex vivo* d'une séquence d'ADN transposable d'intérêt (séquence nucléotidique exogène d'intérêt) dans une séquence d'ADN cible. Par exemple, l'invention propose un procédé de préparation d'un médicament, comprenant au moins une étape de transposition (e.g., *in vitro* ou *ex vivo*) d'une séquence d'ADN transposable d'intérêt dans une séquence d'ADN cible, ladite transposition étant médiée par au moins l'un des moyens de l'invention. Le médicament peut ainsi être préparé *ex vivo* si la transposition est réalisée *in vitro.*

Les moyens proposés dans le cadre de la présente invention peuvent par exemple permettre de modifier des cellules afin d'exprimer une protéine médicament (i.e., une protéine d'intérêt thérapeutique ou prophylactique, par exemple l'insuline, un anticorps particulier, etc.). Ces moyens peuvent également permettre de « corriger » des cellules afin de restaurer une fonction biologique déficient. Selon encore un autre mode de réalisation, l'un au moins de ces moyens est utilisé pour la mutagénèse insertionnelle, ou encore pour le séquençage et/ou le clonage d'acides nucléiques.

Ces applications impliquent d'une manière générale la mise en oeuvre d'une transposition *in vitro* ou *in vivo* (notamment dans des cellules hôtes de plante) dans une cellule eucaryote ou prokaryotes à l'exception d'une cellule dans un organisne humain ou animal, ce qui relève des connaissances générales de l'homme du métier dans le domaine de l'invention (Ausubel *et al*., 1994 ; Craig *et al.,* 2002). Concernant plus particulièrement les transpositions *in vivo,* la séquence d'ADN cible est typiquement le génome de l'hôte, qui peut être un organisme, eucaryote (par exemple une cellule hôte de plante) ou procaryote, ou un tissu d'un organisme, ou encore une cellule d'un organisme ou d'un tissu à l'exception d'un organisme humain ou animal ou d'une cellule ou d'un tissu dans un organisme humain ou animal.

En tous les cas, les nombreuses applications des moyens de l'invention, pour lesquelles ceux-ci se révèlent d'un intérêt considérable, font appel à des techniques conventionnelles de biologie moléculaire bien connues de l'homme du métier.

Egalement décrite est l'utilisation d'une transposase Mos-1 hyperactive comprenant au moins :
- une mutation au niveau d'au moins un résidu choisi parmi les résidus suivants de la séquence SEQ ID N°2 : F53, Q91 et Y237, et/ou
- la mutation T216A.
pour améliorer, d'un facteur au moins égal à 5, de préférence au moins égal à 10, la fréquence de transposition d'une séquence nucléotidique exogène d'intérêt portée par un pseudo-transposon *Mos-1* dans lequel ladite séquence nucléotidique exogène d'intérêt remplace la séquence nucléotidique codant la transposase Mos-1 d'origine.

L'utilisation d'une telle transposase se fait notamment dans un système de transposition recombinant hyperactif tel que décrit supra.

En particulier, ladite transposase Mos-1 hyperactive comprendra au moins une mutation choisie parmi les mutations F53Y, Q91R, T216A, Y237C, et leurs combinaisons. Elle pourra en outre comprendre une mutation au niveau du résidu E137, notamment la mutation E137K, à l'exclusion de la combinaison de mutations Q91R+E137K+T216A ou F53Y+E137K+T216A.

En pratique, la transposase Mos-1 hyperactive sera généralement codée par une séquence nucléotidique placée sur un vecteur, sous le contrôle d'éléments de régulation de l'expression. L'expression de la transposase sera ainsi avantageusement inductible.

Conformément à la description qui précède, la transposase Mos-1 hyperactive fait de préférence partie d'un système de transposition recombinant hyperactif, dans lequel elle est fournie en *trans* d'un pseudo-transposon *Mos-1* tel que décrit plus haut. En particulier, la séquence nucléotidique exogène d'intérêt contenue dans le pseudo-transposon *Mos-1* est un gène fonctionnel. Par ailleurs, au moins l'une des deux répétitions terminales non traduites (UTR) et/ou au moins l'une des deux répétitions terminales inversées (ITR) dudit pseudo-transposon *Mos-1* est(sont) génétiquement modifiée(s). Il sera en pratique avantageux d'utiliser un pseudo-transposon *Mos-1* porté par un vecteur.

Les figures suivantes sont fournies à titre purement illustratif et ne limitent en aucune façon l'objet de la présente invention.
Figue 1 : Séquence nucléotidique du gène de la transposase du transposon *mos-1* (SEQ ID N°1) et séquence protéique de la transposase Mos-1 (SEQ ID N°2). Les sites d'hyperactivité (sites cibles de la mutagénèse dirigée) sont localisés par un cadre simple autour des résidus qui apparaissent sur fond gris. Les sites putatifs de phosphorylation sont localisés de la manière suivante :
- cadre double : acide aminé phosphorylable par les ATM kinases ;
- cadre hachuré gras : acide aminé phosphorylable par la protéine kinase C (pKc) ;
- cadre gris clair épais : acide aminé phosphorylable par la protéine kinase AMPc dépendante (pKa) ;
- cadre noir épais: acide aminé phosphorylable par la pKa et la protéine kinase GMPc dépendante (pKg) ;
- cadre hachuré simple : acide aminé phosphorylable par la caséine kinase II (CKII).
Les résidus QTQ (positions 87 à 89 de la séquence protéique SEQ ID N°2) correspondent au site putatif de phosphorylation par la famille des ATM kinases. Les cercles sur fond pointillé marquent les résidus fortement phosphorylables.
Les cercles sur fond gris identifient les résidus impliqués dans la triade catalytique caractéristique des transposases de MLE (D,D34-35[D/E]) et impliqués dans la coupure de l'ADN.
Les flèches verticales indiquent les sites de clivage protéolytique.

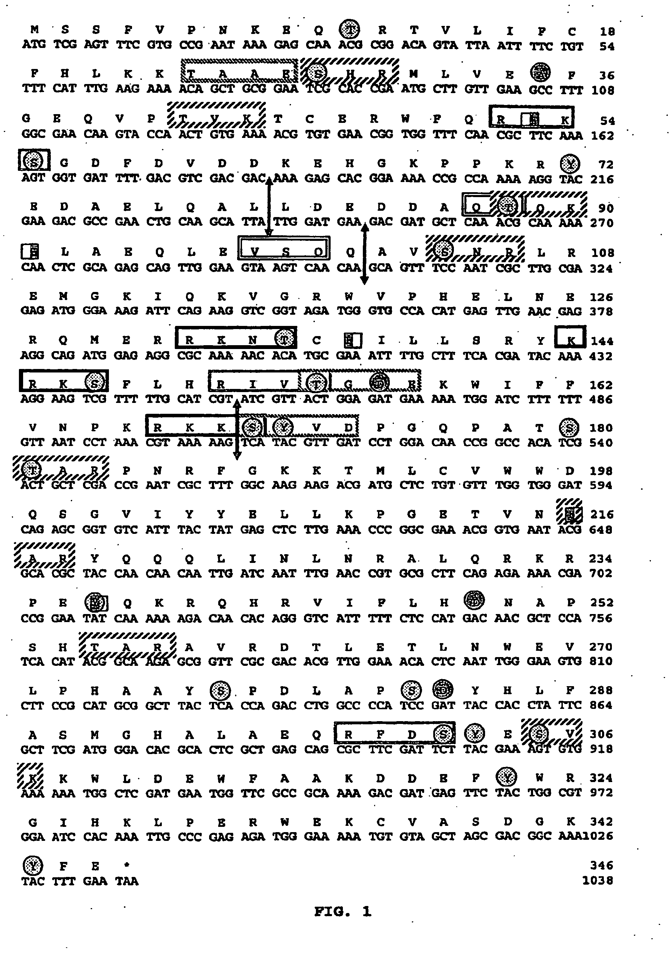

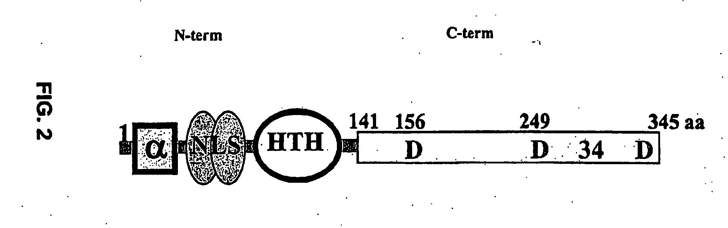

Figure 2 : Schéma représentant la structure de la transposase de l'élément *Mos-1.*
N-term : domaine N-terminal responsable de la liaison aux ITR ;
C-term : domaine C-terminal responsable de la catalyse du transfert des brins d'ADN ; NLS: signal putatif de localisation nucléaire (ou internationalisation nucléaire); HTH : motif hélice-tour-hélice; aa : acide aminé.
Les nombres indiquent les positions des acides aminés.
La triade catalytique caractéristique [D, D34 (D/E)] est signalée.
Figure 3 : Représentation schématique du plasmide pBC3Neo3, construit à partir du plasmide pBC SK+ (Stratagene), et de ses dérivés (A et B).

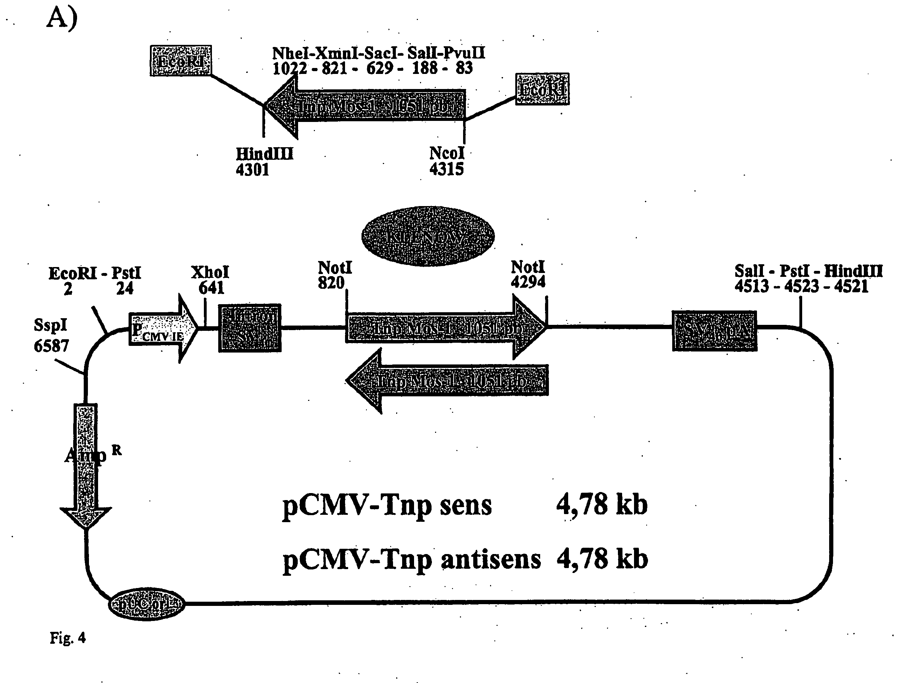

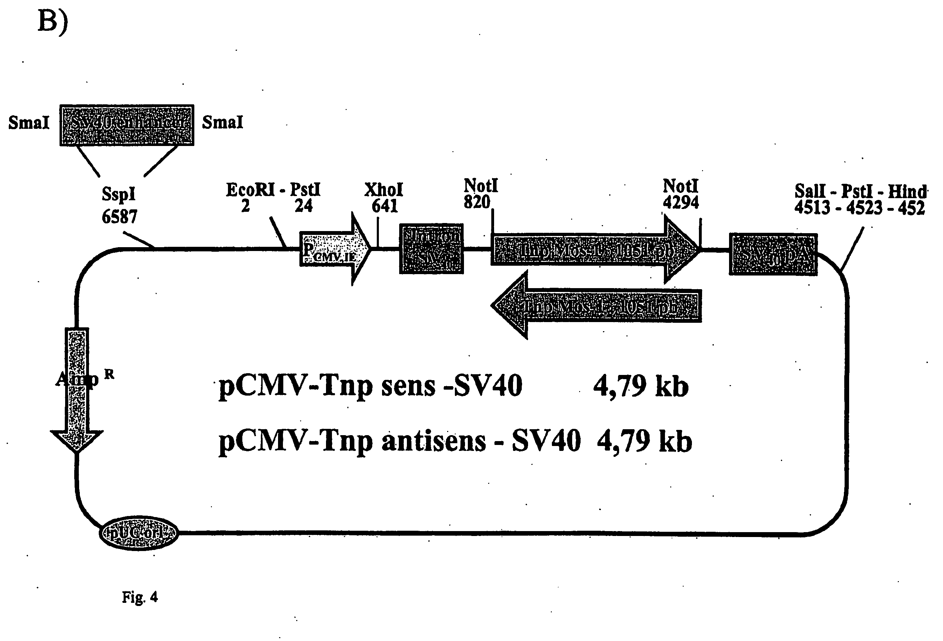

Figure 4 : Représentation schématique du plasmide pCMV-Tnp et de ses dérivés (A et B).

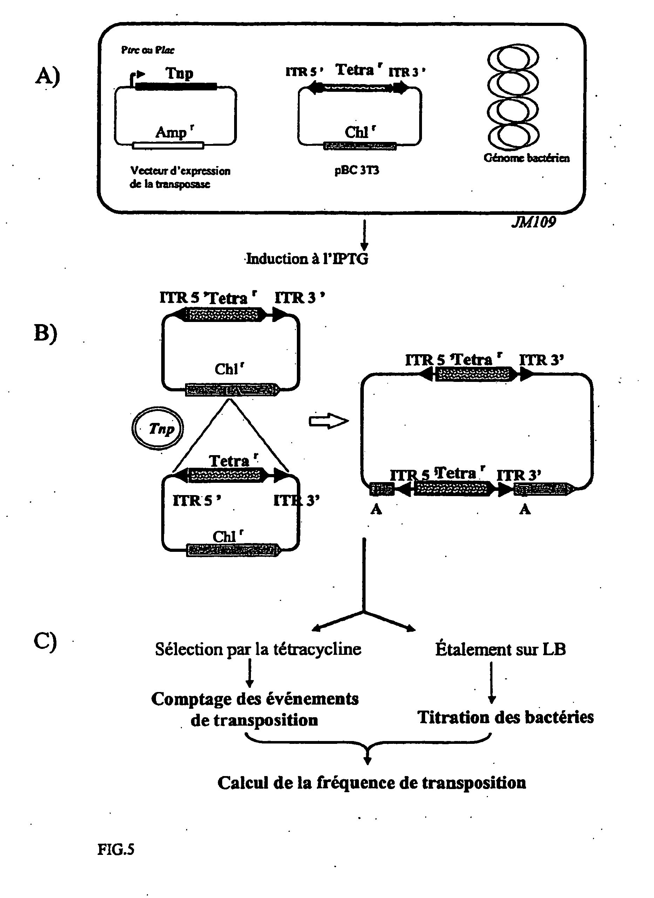

Figure 5 : Représentation schématique du test de transposition.
A) : bactérie co-transformée avec le vecteur d'expression codant la transposase (Tnp) et le vecteur rapporteur de la transposition.
B) : Événement de transposition après induction du vecteur d'expression.
C) : Détermination de la fréquence de transposition.
Figure 6 : Efficacité de transposition des transposases mutantes : facteur d'hyperactivité et fréquence de transposition des mutants.
i) Mutants simples :
   - A t = 0h après induction : A) Facteur d'hyperactivité ; B) Fréquence de transposition;
   - A t = 5h après induction : C) Facteur d'hyperactivité ; D) Fréquence de transposition ;
ii) Mutants multiples :
   - A t = 0h après induction : E) Facteur d'hyperactivité ; F) Fréquence de transposition ;
   - A t = 5h après induction : G) Facteur d'hyperactivité ; H) Fréquence de transposition ;
   WT(53) : transposase sauvage.

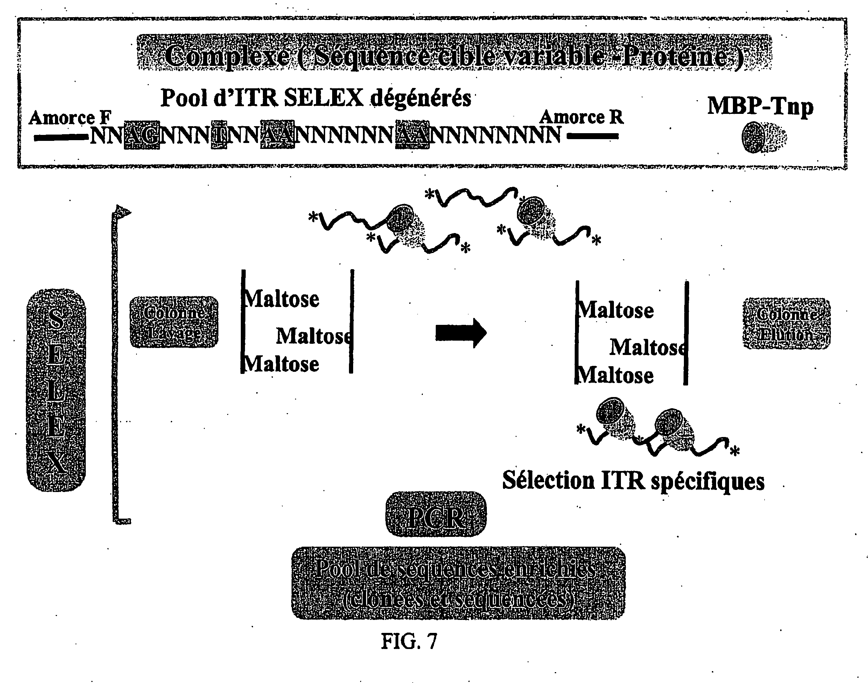

Figure 7 : Schéma illustrant le principe général de la méthode SELEX.

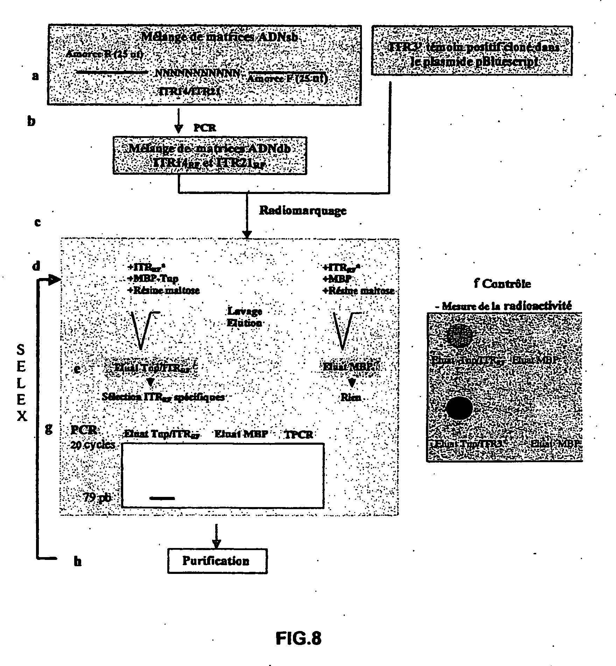

Figure 8 : Schéma illustrant le principe de la méthode SELEX 1.

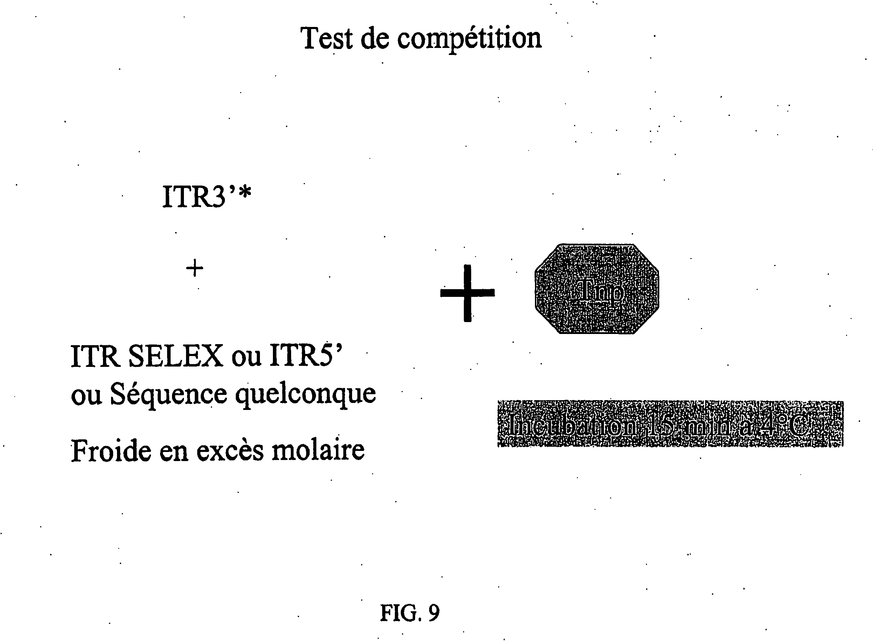

Figure 9 : Schéma présentant les tests de compétition.

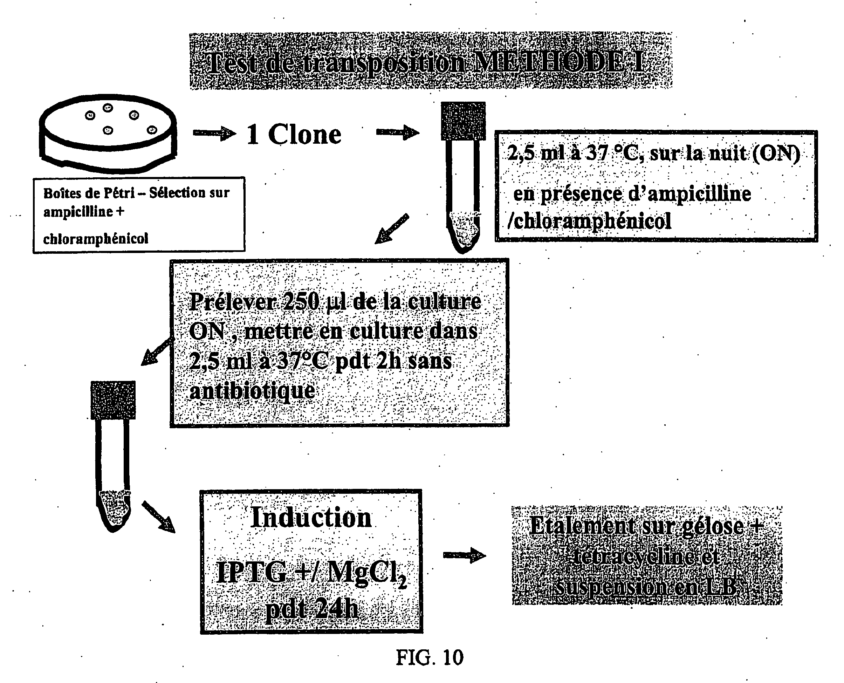

Figure 10 : Schéma représentant les conditions opératoires des tests de transposition *in vivo* en bactéries selon la méthode I.

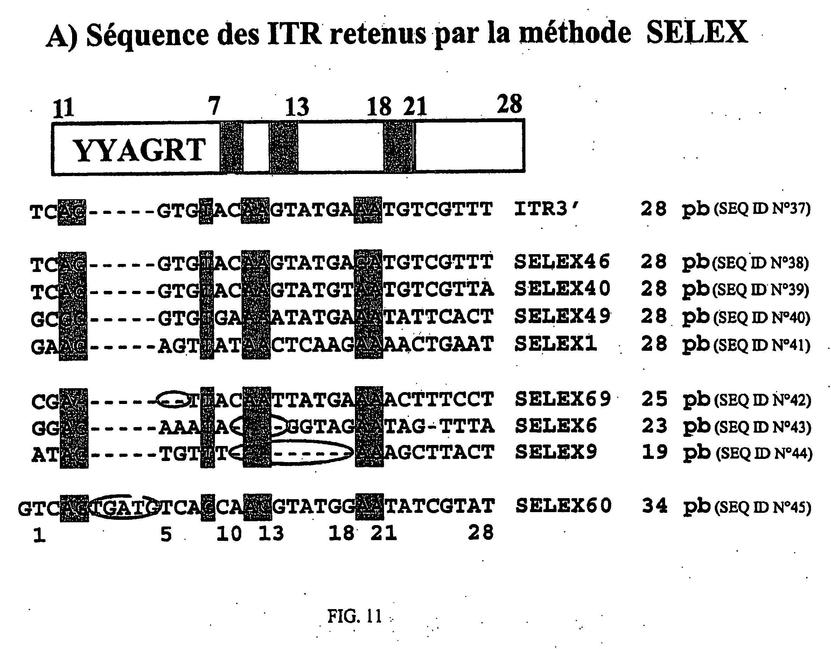

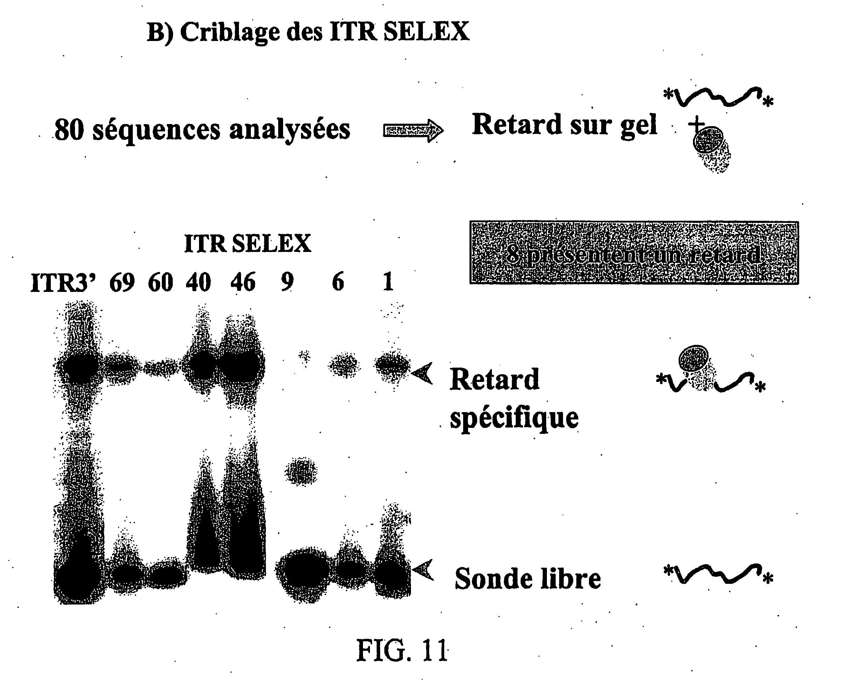

Figure 11 : Résultats des gels retards (B) réalisés avec les ITR présentés en (A) (ITRSelex sélectionnés par les méthodes SELEX mises au point par les Inventeurs).

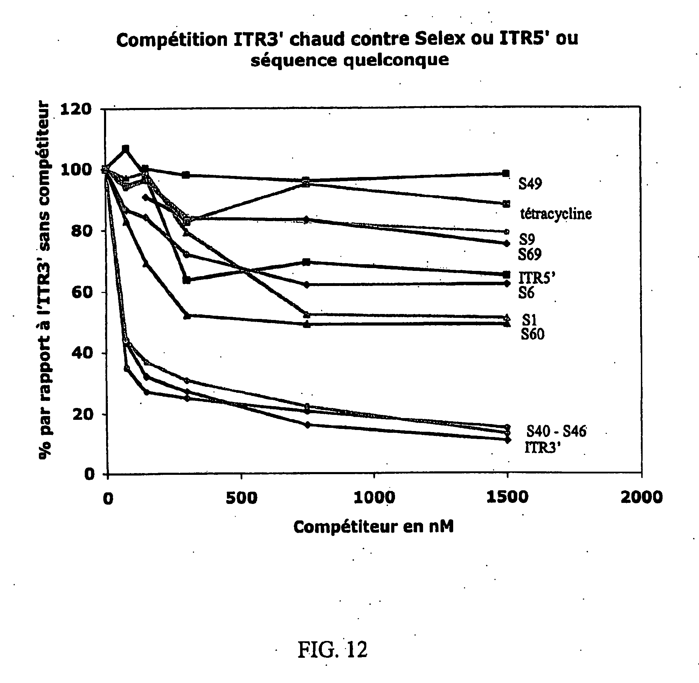

Figure 12 : Résultats des tests de compétition.

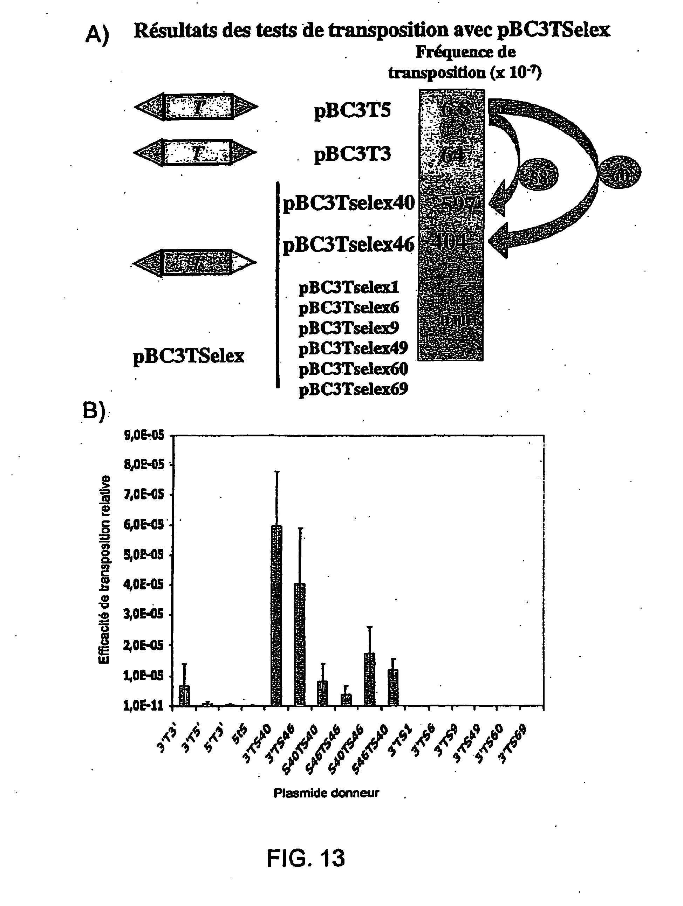

Figure 13 : A) Résultats de tests de transposition obtenus avec les ITR et B) Résultats complémentaires de tests de transposition en bactérie avec les ITRSelex et la transposa se Mos-1 sauvage.

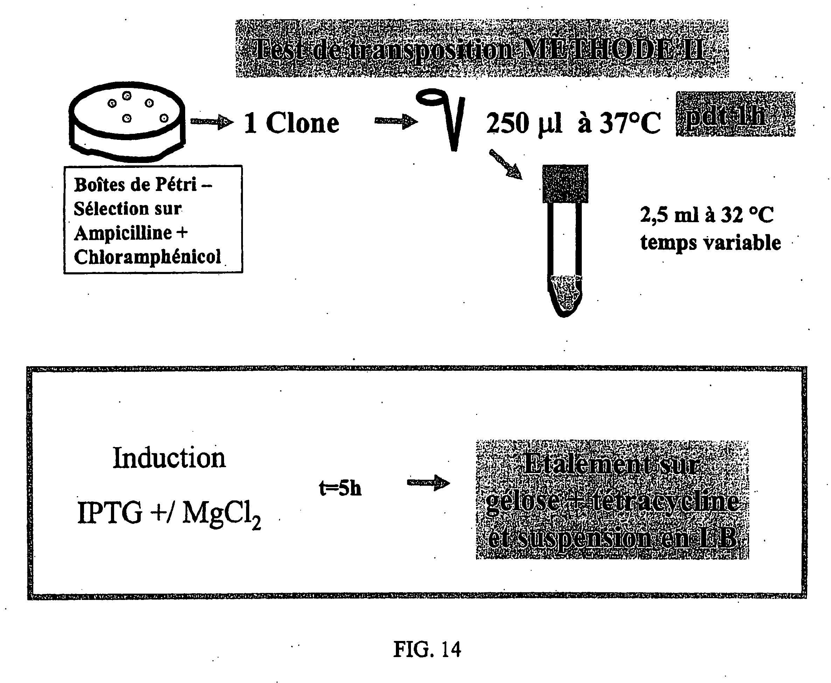

Figure 14 : Schéma illustrant les conditions opératoires de la méthode II de transposition *in vivo* en bactéries.

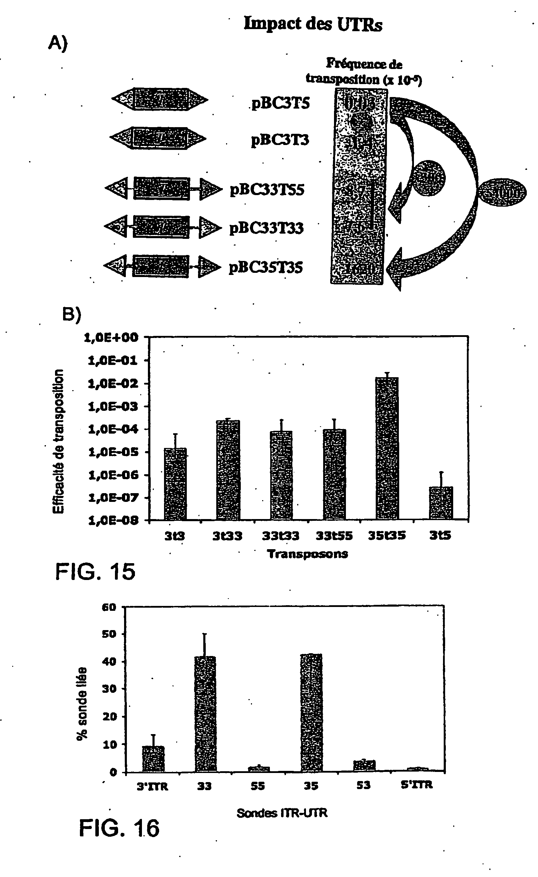

Figure 15 : A) Résultats de tests de transposition obtenus avec les ITR/UTR et B) Résultats complémentaires de tests de transposition en bactérie avec les combinaisons ITR/UTR et la transposase Mos-1 sauvage.
Figure 16 : Représentation graphique de la quantité de complexes [Transposase Mos-1 sauvage + ITR] formés avec des combinaisons ITR/UTR.

La partie expérimentale ci-après, appuyée par des exemples et des figures, illustre de manière non limitative l'invention.

### EXEMPLES

### PARTIE I: TRANSPOSASES MOS-1 MUTANTES HYPERACTIVES (Tnp)

### I- MATERIEL ET METHODES

### I-A- Vecteurs utilisés

### I-A-1 Description des plasmides utilisés

Le vecteur pGEM-T-Easy (3,1 kb) (Promega Charbonnières France ; cat. #A1360) possède les amorces de séquençage Pu et Prev (Ausubel et al, 1994) et le gène de résistance à l'ampicilline. Il a été conçu pour cloner des produits de PCP dans le gène LacZ, ce qui permet un criblage blanc/bleu des colonies bactériennes obtenues sur boîte LB ampicilline, en présence de X-Gal et d'IPTG. Il a été utilisé pour donner le pGEM-T (Tnp) (Augé-Gouillou et al, 2001). Ce dernier sert de matrice pour la mutagenèse de la transposase avant son sous-clonage dans les vecteurs pKK-233-2 et pCS2+.

Le vecteur pKK-Tnp (5,6 kb) permet une expression forte de la transposase via un promoteur P*lac* inductible à l'IPTG. Le promoteur n'est pas modulable et présente une fuite d'expression naturelle. Le pKK-Tnp porte également te gène de résistance à l'ampicilline. Ce plasmide est dérivé du vecteur pKK-233-2 (Clontech, Ozyme, Saint Quentin en Yvelines, France).

Le vecteur pMaIC2x-Tnp, dérivé du pMalC2x (New England Biolabs, Ozyme, Saint Quentin en Yvelines, France), permet une expression de la transposase fusionnée en N-terminal à la protéine MBP (Maltose binding protein). Il porte le gène de résistance à l'ampicilline.

Le vecteur pCS2+-Tnp est dérivé du vecteur pCS2+ (Tumer DL et al., 1994) et permet une expression de la transposase en cellules eucaryotes sous le contrôle du promoteur CMVie. Ce vecteur permet également de synthétiser des ARN *in vitro* correspondant à l'ARN messager codant pour la transposase. La transcription est réalisée sous le contrôle du promoteur SP6 et l'ARN est polyadénylé.

Le pBC 3T3 est un plasmide donneur de pseudo mariner Mos-1 (Augé-Gouillou et al, 2001). Il contient le gène de résistance à la tétracycline « OFF » (c'est à dire sans promoteur) bordé par deux ITR 3'. Ainsi, seules les bactéries qui ont effectué la transposition du pseudotransposon en aval d'un promoteur (promoteur « tagging ») sont sélectionnées sur boîtes LB tétracycine. Le vecteur porte le gène de résistance au chloramphénicol.

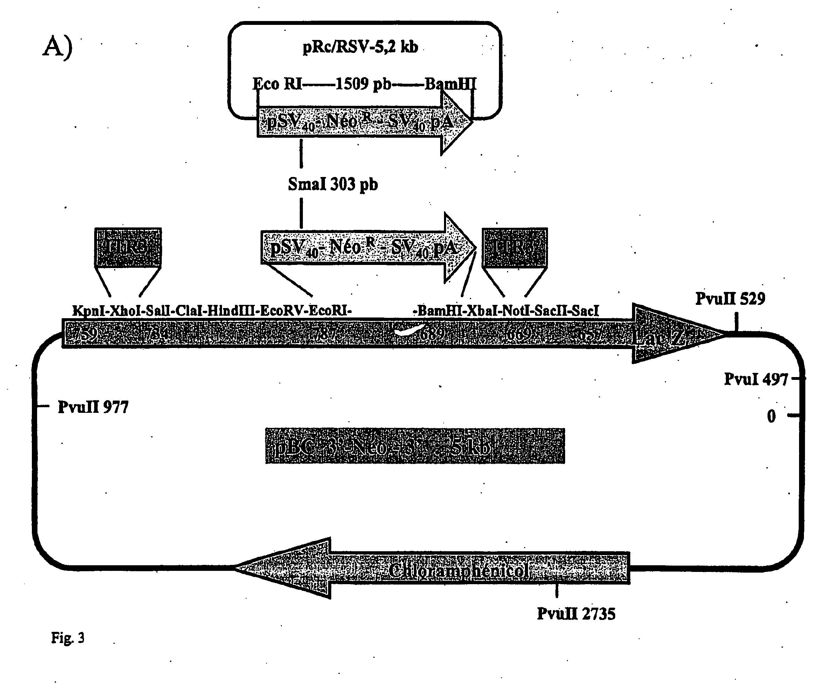

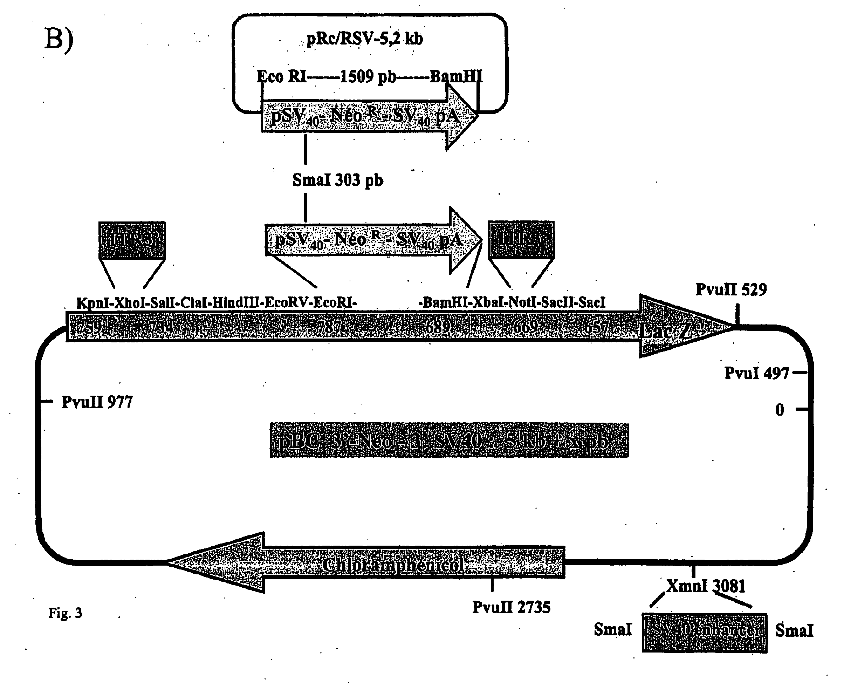

Le pBC3Neo3 (Fig 3) est un plasmide donneur de pseudo mariner Mos-1. Il contient le gène de résistance à la néomycine sous le contrôle du promoteur SV40 bordé de deux ITR 3'. Ceci permet la sélection de cellules eucaryotes dans lesquelles le gène de résistance à la néomycine a été intégré dans le génome de la cellule. La sélection s'effectue à l'aide de G418 (800µg/ml) pendant 2 semaines. Le vecteur porte le gène de résistance au chloramphénicol.

Les plasmides pBC 3T3 e' pBC3Neo3 contiennent donc le pseudo-transposon *Mos-1* dans lequel la répétition terminale inversée sauvage située en 5' (ITR 5') a été mutée de sorte qu'elle est une copie parfaite de la répétition terminale inversée sauvage située en 3' (ITR 3'). Ce pseudo-transposon est donc bordé de 2 ITR 3' (« pseudo-transposon 2 ITR3' »). Ce pseudo-transposon a été utilisé, en association avec la transposase Mos-1 sauvage, pour déterminer la fréquence de transposition de référence, à partir de laquelle ont été déterminés les facteurs d'hyperactivité associés à la mise en oeuvre des systèmes selon l'invention.

De manière générale dans ce type de constructions (pseudo-transposons), la lettre « T » représente le gène rapporteur conférant la résistance à la tétracycline.

Le pBC KS Neo est un plasmide contenant le gène de résistance à la néomycine sous le contrôle du promoteur SV40. Le vecteur porte le gène de résistance au chloramphénicol.

Le vecteur pGL3-Control (Promega; Cat. #E1741) est un plasmide contenant le gène codant pour la luciférase sous le contrôle du promoteur SV40. Il porte aussi le gène de résistance à l'ampicilline.

### I-A-2 Construction des vecteurs

### I-A- 2- 1- Préparation de l'ADN des vecteurs

Pour les différentes constructions, toutes les élutions d'ADN à partir d'un gel d'agarose ont été réalisées avec le kit Wizard SV Gel and PCR Clean-Up system (Promega, France). Tourtes les minipréparations de plasmides à partir de cultures bactériennes ont été effectuées avec le kit Wizard Plus minipréps (Promega). Les préparations à plus grande échelle d'ADN ont été réalisées avec le kit Pureyield plasmid midiprep system (Promega) ou avec les kits Midiprep ou Maxiprep (Qiagen).

### I-A-2- 2- Mutagenèse dirigée pour l'obtention des mutants.

La mutagenèse dirigée a été réalisée d'après le protocole du kit Quikchange site directed mutagenesis (Stratagene). Les oligonucléotides permettant d'introduire la mutation ont été synthétisés par MWG Biotech (Roissy CDG). Les enzymes Pfu polymérase et Dpn1 ont été fournies par Promega (France). Brièvement, la mutation à introduire est portée par deux oligonucléotides complémentaires. La totalité du plasmide a été amplifiée par PCR (95°C 1 min puis 16 cycles 95°C 30 sec, 55°C 1min, 68°C 2 min/kb de plasmide). Le plasmide ayant servi de matrice à la PCR a été digéré par Dpn1 (1h 37°C, 2-3u/50µl de PCR). 2-3 µl de la PCR traitée par Dpn1 ont ensuite été transformés dans des bactéries XL1Blue chimiocompétentes ou JM109 électrocompétentes.

Les séquences des oligonucléotides utilisés pour la mutagénèse dirigée sont indiquées dans le Tableau 1 suivant et la mutation est précisée par les nucléotides en gras.

**Tableau 1**

| Mutation | Amorce directe | SEQ ID N° | Amorce inverse | SEQ ID N° |
|---|---|---|---|---|
| F53Y | | 3 | cgtcaaaatcaccactttt**gta**gcgttgaaaccacc | 4 |
| Q91R | gctcaaacgcaaaaa**cga**ctcgcagagc | 5 | gctctgcgag**tcg**tttttgcgtttgagc | 6 |
| L92A | cgcaaaaacaa**gcc**gcagagcagttgg | 7 | ccaactgctctgc**ggc**ttgtttttgcg | 8 |
| L92R | cgcaaaaacaa**cgc**gcagagcagttgg | 9 | ccaactgctctgc**gcg**ttgtttttgcg | 10 |
| Q100N | gcagttggaagtaagta**a**ccaagcagtttcc | 11 | ggaaactgcttg**gtt**acttacttccaactgc | 12 |
| Q100R | gcagttggaagtaagtc**ga**caagcagtttcc | 13 | ggaaactgcttg**tcg**acttacttccaactgc | 14 |
| Q100E | gcagttggaagtaagt**gaa**caagcagtttcc | 15 | ggaaactgcttg**ttc**acttacttccaactgc | 16 |
| S104P | | 17 | | 18 |
| N105A | caagcagtttcc**gca**cgcttgcgag | 19 | ctcgcaagcg**tgc**ggaaactgcttg | 20 |
| E137K | ggcgcaaaaacacatgc**aaa**attttgctttcacg | 21 | cgtgaaagcaaaat**ttt**gcatgtgtttttgcgcc | 22 |
| T216A | gcgaaacggtgaat**gcg**gcacgctacc | 23 | ggtagcgtgc**cgc**attcaccgtttcgc | 24 |
| Y237C | | 25 | | 26 |
| W268F | cgttggaaacactcaat**ttc**gaagtgcttccgc | 27 | gcggaagcacttc**gaaa**ttgagtgtttccaacg | 28 |
| W268Y | cgttggaaacactcaat**tac**gaagtgcttccgc | 29 | gcggaagcacttc**gtaa**ttgagtgtttccaacg | 30 |
| W268A | cgttggaaacactcaat**gcg**gaagtgc | 31 | gcacttc**cgca**ttgagtgtttccaacg | 32 |

### I-A-2-3- Vérification de la séquence des transposases.

L'introduction de mutations dans la transposase clonée dans le plasmide pGEM-T easy a été vérifiée par séquençage. Pour cela, 10 microlitres d'une minipreparation d'ADN ont été envoyés pour séquençage à la société MWG Biotech. Les amorces utilisées (Puniv -21 et Prev -49) ont été fournies par cette société.

### I-A-2-4- Sous-clonage des transposases mutantes dans le plasmide pKK-233-2.

Le fragment codant pour la transposase sauvage ou mutante (Tnp) a été préparé à partir du vecteur pGEM-T (Tnp) par digestion Nco1/HindIII et élué sur gel 0,8% agarose (TAE1X : 0,04M Tris-Acétate, 1mM EDTA pH8). Le plasmide pKK-233-2 a été digéré par HindIII /Nco1, déposé sur gel d'agarose, élué puis ligaturé avec le fragment codant pour la transposase de Mos-1 (dite Tnp), pendant une nuit à 16°C. Un contrôle de recircularisation du plasmide sur lui-même a été réalisé en effectuant une ligature du plasmide en absence de fragment codant pour la transposase.

Le produit de ligature a été utilisé pour transformer des bactéries *E. coli* JM109 qui ont ensuite été sélectionnées sur boîte LB-ampicilline (100 µg/ml). 4 clones résistants à l'ampicilline ont été mis en culture pour une extraction du plasmide. Les mini-préparations d'ADN ont été contrôlées par digestion EcoR1/HindIII puis en électrophorèse sur gel 0,8% agarose (TAE 1X) afin de s'assurer qu'ils avaient Intégré le gène codant pour la transposase.

### I-A-2-5- Sous-clonage des transposases mutantes dans le plasmide pMaIC2X.

Pour le sous-clonage en pMaIC2X, le gène codant pour la transposase a dû être réamplifié par PCR à l'aide des amorces MTP up et 3' HindIII:
MTP up : 5'-TACGTAATGTCGAGTTTCGTGCCG (SEQ ID N°33)
3'HindIII : 5'-CCCAAGCTTATTCAAAGTATTTGC (SEQ ID N°34) Conditions de cycle : 95°C 5 min puis 20 cycles (95°C 30sec, 50°C 1min, 72°C 1 min) puis 72°C 5 min.

Le produit de PCR a ensuite été déposé sur gel, élué dans 50 µl et cloné dans le plasmide pGEM-T easy (1 µl de vecteur + 2 µl de produit de PCR élué). Après ligature une nuit à 16°C, le produit de ligation a été transformé en cellules JM109 par électroporation et les bactéries ont été sélectionnées sur boîte LB Ampicilline (100µg/ml) contenant du X-Gal 2% IPTG 1mM. Deux clones blancs résistants à l'ampicilline ont été mis en culture pour extraire l'ADN plasmidique. Après contrôle du clonage par digestion EcoR1 et électrophorèse sur gel d'agarose 0,8% (TAE 1X), le plasmide a été envoyé pour séquençage à la société MWG Biotech.

Après vérification de la séquence, le fragment codant pour la transposase a été préparé par digestion SnaB1/HindIII et élué sur gel. Il a été ligaturé avec le plasmide pMalC2X ouvert par Xmn1/HindIII. Des bactéries JM109 ont ensuite été transformées par le produit de ligation et étalées sur boites LB Ampicilline (100µg/ml).

### I-A-2-6- Sous-clonage des transposases mutantes dans le plasmide pCS2+

Le fragment codant pour la transposase sauvage ou mutante (Tnp) a été préparé à partir du vecteur pGEM-T (Tnp) par digestion EcoR1 et élué sur gel 0,8% agarose (TAE1X: 0,04M Tris-Acétate, 1mM EDTA pH8). Le plasmide pCS2+ a été ouvert par EcoR1, dephosphorylé puis ligaturé avec le fragment contenant la transposase. Le produit de ligature a été transformé dans des bactéries JM109 et les clones ont été sélectionnés sur boîte LB Ampicilline (100µg/ml). Huit clones ont été mis en culture pour extraire l'ADN plasmidique. La présence de l'insert et son orientation a été évaluée par digestion PvuII/BamH1 ou PvuII seul et électrophorèse sur gel d'agarose 0,8% en TAE 1X. Les clones sont désignés sens + quand le gène est inséré dans le sens permettant la transcription de l'ARNm correspondant à la transposase sous le contrôle du promoteur SP6. Les clones sont désignés sens - quand le gène est inséré dans le sens contraire à la transcription sous le contrôle du promoteur SP6.

### I-B- Analyse de l'activité des transposases mutantes en bactéries :

### Test de transposition en bactéries

Des bactéries *Escherichia coli* JM109 ont été co-transformées avec le plasmide pBC 3T3 (porteur d'un pseudo-mariner 2 ITR 3' rapporteur de la transposition et du gène de résistance au chloramphénicol) et avec le vecteur inductible codant pour la transposase (pKK-Tnp ou pMal-Tnp) porteur du gène de résistance à l'ampicilline). La sélection des bactéries a été effectuée sur ampicilline (100µg/ml) et chloramphénicol afin de vérifier la présence des deux plasmides.

Les bactéries JM109 contenant à la fois le plasmide pBC 3T3 et le plasmide pKK-Tnp (ou pMalC2X-Tnp) ont été mises en culture 1h à 37°C dans 250 µl de LB. Cet inoculum a ensuite été versé dans 5 ml de LB supplémenté en IPTG 1 mM. La culture a été titrée sur boîte LB (100 µl d'une dilution au 1/1000) et sur boîte LB-Tet (12,5 µg/ml) (250 µl de la culture non diluée). La culture induite par l'IPTG a ensuite été cultivée 5 heures à 32°C (température optimale de la transposase) sous agitation (250rpm). La suspension bactérienne a été ensuite titrée sur boîte LB (100 µl d'une dilution au 1/250000) et sur boîte LB-Tet (100 µl de la suspension bactérienne pure). Les boîtes ont été placées à 37°C pour la nuit. Le lendemain, les colonies ont été comptés sur les boîtes LB et LB-Tet, puis on a calculé la fréquence de transposition (égale au nombre de bactéries résistantes à la tétracycline sur le nombre de bactéries pour 1 ml de suspension bactérienne pure).

### I-C- Analyse de l'activité des transposases mutantes en cellules eucaryotes

### I-C-1- Transfection cellulaire

Le jour précédant la transfection, 2.10⁵ cellules HeLa ont été réparties par puits de plaques 6 puits. Le lendemain, les cellules ont été transfectées avec 3 µg d'ADN (750 ng de pGL3-Control, 750 ng de pCS2/Tnp, 1500 ng de pBC3T3) et du PEI (ratio 1/10) (Eurogentec). Chaque condition a été testée en double.

Deux jours après la transfection, l'efficacité de transfection a été évaluée en lysant les cellules et en évaluant l'activité luciférase. Les cellules du second puits ont été trypsinées et mises en culture dans deux boîtes de culture de 10 cm de diamètre en présence d'agent de sélection G418 (800 µg/ml). Le milieu de culture a été changé tous les deux jours et la pression de sélection a été maintenue pendant quinze jours. 5 clones par condition ont été isolés et amplifiés pendant 15 jours en pression de sélection (200µg/ml de G418).

### I-C-2- Analyse moléculaire des clones

L'ADN génomique des différents clones est extrait puis soumis à une analyse par Southern Blot après restriction enzymatique. Cette méthode permet d'analyser les différents sites d'insertion de la cassette de résistance au G418. L'analyse par séquençage permet de vérifier la présence de la duplication du dinuctéotide TA qui signe les événements de réelle transposition et d'évaluer la fréquence des événements de recombinaison aléatoire et transposase dépendante.

### II- RESULTATS

Pour pouvoir analyser toutes les étapes de la transposition de Mariner Mos-1 dans un système plus simple que les cellules eucaryotes, un modèle d'étude en bactéries a été mis au point. L'utilisation de ce système a permis d'évaluer, à différents temps de transposition, l'efficacité de transposition des différentes transposases mutantes.

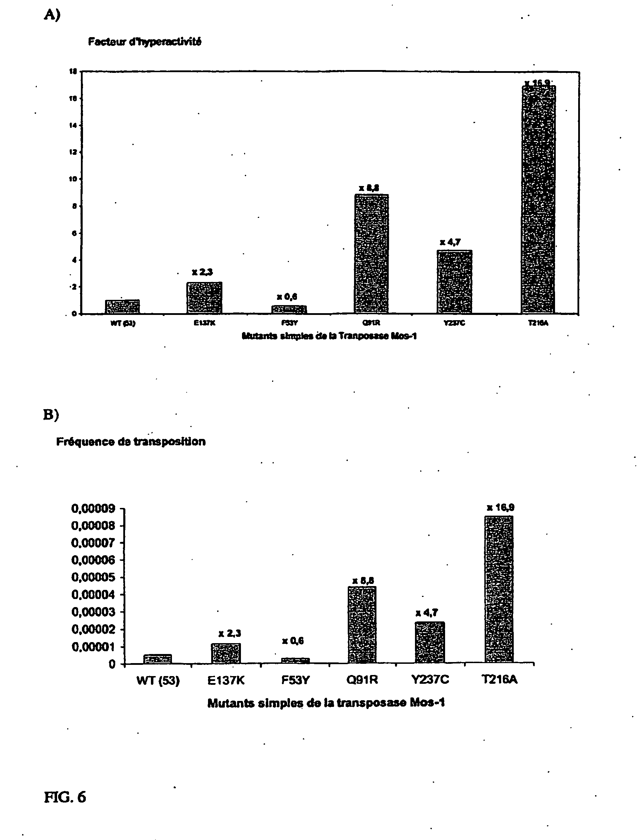

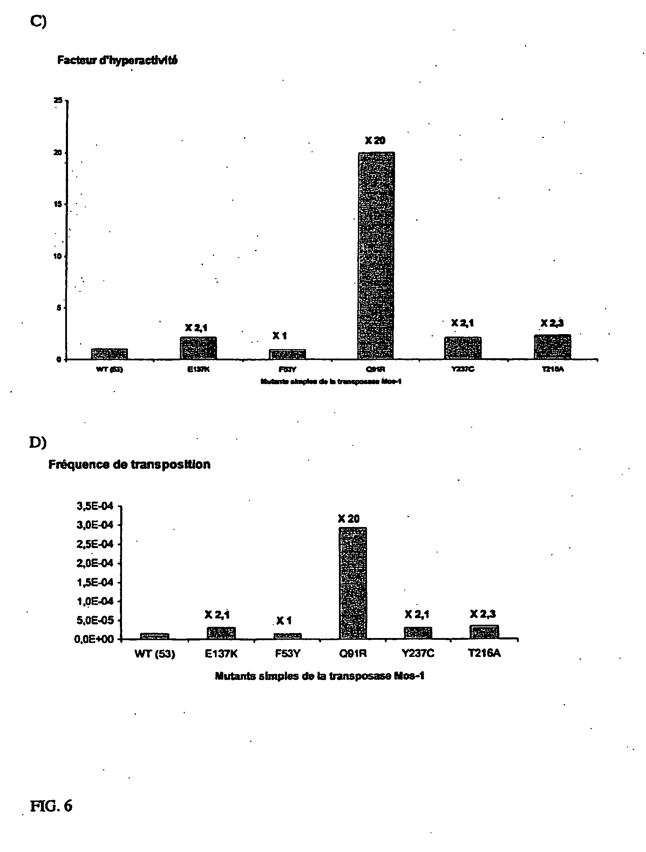

Les résultats obtenus pour les mutations simples sont présentés dans la figure 6A et 6B pour un temps de transposition T0 après l'induction. Les résultats sont exprimés en médiane de facteur d'augmentation par rapport à la transposase sauvage. Les mutations les plus intéressantes sont les mutations situées sur les positions E137, Q91, Y237, T216. La même analyse, mais pour un temps de transposition T=5h après l'induction, a donné les résultats illustrés par la figure 6C et 6D.

Les mutants multiples ont été réalisés en associant les mutations les plus prometteuses. Sept doubles mutants, trois triples, deux quadruples et 1 quintuple ont été obtenus, conformément au Tableau 2 ci-dessous.

**Tableau 2**

| | |
|---|---|
| Doubles mutants | F53Y + T216A |
| | F53Y + Y237C |
| | F53Y + Q91R |
| | Q91R + Y237C |
| | E137K + T216A |
| | E137K+ Y237C |
| | T216A + Y237C |
| Triples mutants | Q91R + E137K + T216A |
| | F53Y + E137K + T216A |
| | F53Y + T216A + Y237C |
| Quadruples mutants | F53Y + E137K + T216A + Y237C |
| | F53Y + Q91R + T216A + Y237C |
| Quintuple mutant | F53Y + Q91 R + E137K + T216A + Y237C |

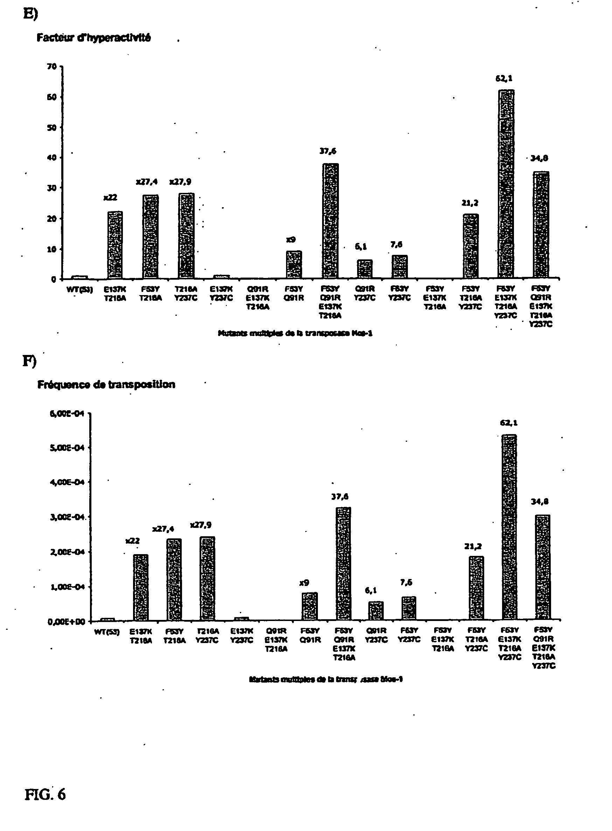

Les résultats des tests de transposition réalisés avec les mutants multiples sont reportés dans la figure 6E à 6H.

Certaines associations (Q91E E137K T216A; F53Y E137K T216A) entraînent une perte complète de transposition. Les autres combinaisons améliorent la transposition, d'au moins un facteur 6 pour le temps To (figure 6E). Les combinaisons les plus intéressantes sont les associations F53Y Q91R E137K T216A, F53Y E137K T216A Y237C et le quintuple mutant (Figures 6E et 6F).

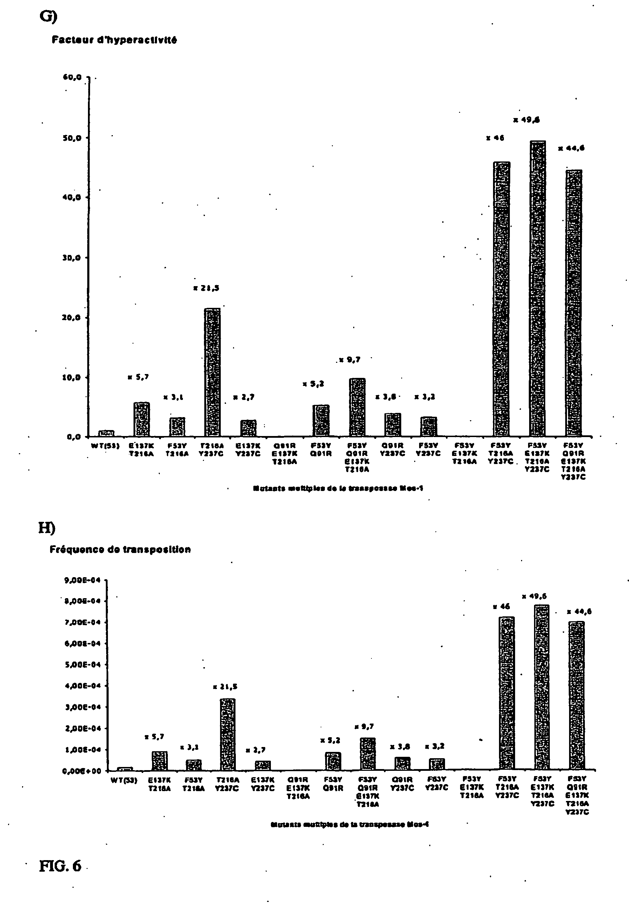

Des résultats similaires ont été obtenus pour un temps de transposition de 5 heures (Figures 6G et 6H).

On notera que des mutations, par ailleurs décrites dans la littérature comme ayant un effet sur certaines propriétés de la transposase Mos-1, ne sont pas pour autant intéressantes lorsqu'il s'agit d'identifier des transposases Mos-1 mutantes hyperactives. C'est le cas, par exemple, de la mutation S104P, rapportée dans Zhang et al. (2001) comme modifiant la capacité de la transposase Mos-1 à créer des interactions protéiques. Dans le cadre de leurs travaux, les inventeurs ont en effet pu observer que cette mutation entraînait une abolition totale de l'activité de transposition de la transposase Mos-1 lors de la mise en oeuvre des tests de transposition en bactéries (données non montrées).

### PARTIE II: PSEUDO-TRANSPOSONS Mos-1 RECOMBINANTS HYPERACTIFS

### 1- Les ITR (Inverted Terminal Repeat)

Brièvement, la recherche de séquences d'ITR optimisées a été réalisée par la technique SELEX qui consiste à obtenir par combinatoire un ensemble d'ITR (Figure 7). Seules certaines positions connues pour être essentielles au bon fonctionnement de la transposase ont été conservées. La nature des autres nucléotides a donc varié de façon aléatoire. Les différents ITR ont été sélectionnés et enrichis pour leur capacité à fixer la transposase. Parmi les ITR sélectionnés, seuls certains étaient capables de retarder la transposase en gel retard (technique EMSA). La transposase associée aux ITR modifiés a été testée en test de transposition en bactérie pour évaluer l'impact du changement de séquence des ITR sur l'efficacité de transposition. Pour certaines configurations (qui affectent par exemple un ou deux nucléotides par rapport à la séquence sauvage), l'efficacité de la transposition a été améliorée d'un facteur significatif, notamment d'un facteur au moins égal à 5.

### I- 1- Matériel et méthodes

### a) Mise au point de la méthode SELEX

L'utilisation de la technique SELEX a permis aux Inventeurs de sélectionner des ITR qui présentent une affinité plus grande pour la transposase que les ITR sauvages, afin d'améliorer les performances du pseudo-transposon *Mos-1* recombinant et du système de transposition objets de la présente invention.

La méthode SELEX, décrite en 1990 (Ellington et al., 1990 ; Tuerk et al., 1990), permet de sélectionner des acides nucléiques dans des mélanges contenant plus de 10¹⁵ molécules différentes, en fonction de propriétés particulières, par exemple la capacité de se lier à une protéine. Le principe général de la méthode consiste à incuber une molécule cible particulière avec un mélange de séquences différentes (ARN, ADN simple brin ou double brin). La fraction capable de se lier à la molécule cible est isolée du reste des acides nucléiques par colonne de chromatographie, immunoprécipitation ou toute autre technique de purification appropriée. Par la suite, la fraction enrichie est amplifiée par PCR ou RT-PCR puis utilisée pour un nouveau tour de sélection. La répétition des cycles de sélection et d'amplification permet d'enrichir le mélange initial en oligonucléotides fonctionnels, appelés aussi « aptamers ». Plus on augmente le nombre de cycle de sélection et d'amplification, plus la quantité d'aptamers augmente, jusqu'à dominer dans la population d'oligonucléotides (pour une revue sur la méthode Selex, voir Klug et al., 1994).

Deux méthodes SELEX, décrites ci-dessous, ont été mises au point par les Inventeurs.

### a1) Origine de la transposase

Une protéine recombinante couplant les qualités de fixation des ITR par la transposase (Tnp) et les qualités de fixation du maltose par la protéine de liaison au maltose (MBP : Maltose Binding Protein) a été utilisée. Cette protéine recombinante, produite en bactérie et nommée MBP-Tnp, se lie aux ITR grâce au domaine de liaison spécifique de la transposase localisé en N-terminal et la MBP permet de purifier les complexes ITR/transposase sur une colonne de maltose.

### a2) Nature des ITR de séquences dégénérées

Pour réaliser le SELEX, un mélange d'oligonucléotides de 79 bases a été synthétisé par la société MWG Biotech. La structure générale de ces oligonucléotides comprend la séquence d'un ITR dégénéré de 29 bases, bordée à chacune de ces extrémités par la séquence des amorces R et F de 25 bases chacune. Ces amorces de séquences respectives 5'-CAGGTCAGTTCAGCGGATCCTGTCG-3' (SEQ ID N°35) et 5'-GAGGCGAATTCAGTGCAACTGCAGC-3' (SEQ ID N°36) ont permis, dans les étapes ultérieures des SELEX, d'amplifier par PCR les séquences des ITR sélectionnés.

Deux mélanges distincts d'oligonucléotides ont été synthétisés ; un dont l'ITR de 29 bases est dégénéré sur 14 positions (ITR14), et un dont l'ITR est dégénéré sur 21 positions (ITR21). Les positions conservées à 100 %, chez tous les éléments de la sous-famille *mariner,* étaient maintenues dans les ITR14 et ITR21, tandis que les positions conservées à 60 /80 % n'étaient maintenues que dans les ITR14. Les ITR14 étaient représentés par 2,7x10⁸ séquences, les ITR21 par 4,4x10¹² séquences.

Afin de valider la méthode, l'ITR3' de *Mos-1* a été utilisé comme témoin.

Chacun des ITR14 et ITR21 a été rendu double brin par PCR avant le premier tour de SELEX étant donné que la transposase se fixe sur un ADN double brin.

### a3) Principe de la méthode SELEX 1

Cette méthode est illustrée sur la figure 8. Elle utilise un pool de matrices ADN monocaténaires (sb) formées d'un ITR de 29 nucléotides (nt) dégénéré sur 14 ou 21 positions et de deux amorces R et F de 25 nucléotides qui bordent les extrémités de l'ITR14 et l'ITR21 (a). Les matrices sont rendues double brin (db) par PCR (b). Elles sont ensuite marquées radioactivement (c) puis incubées en solution avec la résine et la protéine de fusion MBP-Tnp (notée Tnp pour simplifier la figure) ou la MBP (d). La réaction d'interaction se déroule pendant 24 heures à 4°C. Après lavage de la colonne, les complexes ADN/protéine sont purifiés grâce à une solution de maltose (e). Les éluats récupérés sont appelés éluats Tnp/ITR lorsque les matrices ont été incubées avec la MBP-Tnp et éluats MBP lorsque les matrices ont été incubées avec la MBP. Pour suivre la sélection après chaque tour de SELEX, un aliquot de chaque éluat est déposé sur une membrane de nylon puis compté (f). Les matrices sélectionnées à chaque tour de SELEX sont amplifiées par PCR (g). Les produits amplifiés sont ensuite contrôlés sur gel d'agarose. Les fragments d'intérêts sont purifiés (h) puis utilisés pour un nouveau tour de sélection.

### * Etape de fixation ADN/protéine :

Afin de suivre la sélection des ITR à chaque tour de SELEX, les séquences nucléotidiques ont été radiomarquées, soit par la T4 polynucléotide kinase, soit par PCR. La sélection des séquences cibles a été effectuée par incubation en solution de la MBP-Tnp, des ITR14 ou des ITR21 radiomarqués, et de la résine de maltose.

Parallèlement, deux expériences témoins ont été réalisées. Un témoin négatif a permis de s'assurer de la spécificité de l'interaction ADN/protéine, en incubant la MBP qui n'a pas d'affinité particulière pour l'ADN, avec les ITR14 ou les ITR21. Le témoin positif a consisté à incuber l'ITR3' avec la MBP-Tnp d'une part et la MBP d'autre part.

### * Etape de lavage et élution :

Après fixation de la protéine sur sa séquence cible, une étape de lavage a été réalisée afin d'éliminer tous les oligonucléotides non liés sans dissocier les complexes. L'élution des complexes retenus a été réalisée en saturant la résine par du maltose. Deux types d'éluats ont été obtenus. L'éluat Tnp/ITR a été produit par élution de la série d'expériences incubant les oligonucléotides cibles avec la protéine recombinante MBP-Tnp. L'éluat MBP a été produit par élution de la colonne mettant en interaction les mêmes ITR cibles avec la MBP.

### * Etape d'amplification des séquences sélectionnées :

L'amplification des ITR sélectionnés a été effectuée directement sur l'éluat Tnp/ITR et l'éluat MBP grâce à la présence des amorces R et F encadrant les séquences ITR (SEQ ID N°35 et 36). Si la sélection était effective, un signal PCR spécifique (de 79 pb) devait être trouvé pour l'amplification des matrices contenues dans l'éluat Tnp/ITR, mais pas pour les matrices de l'éluat MBP (puisque la MBP n'a pas d'affinité pour les ITR). Ce fragment positif a été élué du gel d'agarose et radiomarqué par la T4 polynucléotide kinase. Pour certains cycles de PCR, le marquage a été effectué directement pendant l'étape d'amplification.

Cet amplimère a ensuite été utilisé comme cible enrichie en séquence affine de la Tnp dans le tour de SELEX suivant.

### a4) Principe de la méthode SELEX 2

Des travaux menés par les inventeurs ont montré que l'ITR3' et l'ITR5' ont la même constante de dissociation mais que leur capacité à fixer la transposase est différente. La quantité de protéine active en présence de l'ITR5' est 10 fois plus faible que celle observée en présence de l'ITR3'. Ceci indique que l'ITR3' agit comme un activateur de la capacité de la transposase à lier un ITR. Deux informations sont donc contenues dans un ITR. La première a un effet sur l'activation de la protéine (impact sur le Bmax), la seconde module l'affinité de la transposase pour l'ITR (impact sur le Kd). Afin de tenir compte de ces données, la méthode SELEX 2 a été mise au point par les Inventeurs. Le principe de cette méthode est identique à celui de la méthode SELEX 1. Cependant, les matrices ADN ont été incubées avec la protéine pendant cinq minutes à 4° C avant de réaliser l'accrochage sur la colonne de maltose. Cette méthode devait permettre ainsi de sélectionner des ITR ayant la capacité d'activer et de se lier à la transposase.

### a5) Protocoles

En règle générale, les procédures expérimentales mises en oeuvre par les inventeurs sont basées sur des techniques classiques bien connues de l'homme du métier (Ausubel et al., 1994 ; Sambrook et Russel, 2001).

### (i) Préparation des matrices

### * Synthèse du brin complémentaire

La transposase ne se lie sur l'ADN que sous forme double brin. La synthèse du brin complémentaire des oligonucléotides cibles, ITR14 et ITR21, a été réalisée par extension d'amorce. La réaction a été effectuée à l'identique pour la méthode SELEX 1 et 2.

### * Purification des fragments ADN

Les produits PCR ont été analysés sur gel d'agarose Nusieve 3% (FMC) en tampon TAE 1X. Les fragments d'ADN ont ensuite été purifiés à partir du gel d'agarose afin d'éliminer toute trace de concatémères.

### * Marquage radioactif des ITR14 et ITR21

Le marquage radioactif des séquences a permis de suivre l'évolution de la sélection, à chaque cycle SELEX. Le marquage au [γ³²P]ATP (activité spécifique supérieure à 4500 Ci/mmol) a été effectué avec la polynucléotide kinase du phage T4 (PNK), puis le marquage au [α³² P]ATP (activité spécifique supérieure à 3000 Ci/mmol) a été réalisé par PCR, en présence des amorces R et F (SEQ ID N°35 et 36).

### (ii) Sélection des cibles par la transposase

### * Etape de fixation ADN/protéine

La résine de maltose (New-England Biolabs) a été équilibrée dans le tampon 1 (Tris pH9 20 mM, NaCl 50mM, DTT 1 mM). L'incubation en solution a été effectuée dans un volume final de 1 ml de tampon 1, avec 200 µl de résine auxquels ont été ajoutés successivement 50 µg de protéine MBP-Tnp ou MBP; 200 ng des ITR14, ITR21 ou ITR3'; 2 µg d'ITR 5' comme compétiteur; 5 mM de MgCl₂ et 2 µg d'ADN de sperme de saumon. La réaction d'interaction de la méthode SELEX 1 a été maintenue pendant 24 heures à 4° C sous agitation constante (300rpm). Dans la méthode SELEX 2, les matrices ADN et la protéine MBP-Tnp ou MBP ont été incubées pendant 5 minutes à 4 °C avant d'être passées sur la colonne de maltose, la réaction d'interaction étant maintenue pendant seulement 1 heure à 4 °C.

### * Etape de lavage de la résine et élution des complexes

A la fin de l'incubation, la résine a été lavée, les complexes protéine/ITR élués. Deux élutions successives ont été réalisées affin de récupérer la totalité des complexes.

### (iii) Etape d'amplification des séquences sélectionnées

Après chaque tour de SELEX, les matrices sélectionnées contenues dans les éluats Tnp/ITR et MBP ont été amplifiées avant de servir pour le tour de SELEX suivant.

La réaction de PCR comprenait de 15 à 30 cycles, typiquement 20 cycles (15 cycles en cas d'amplification de fragments parasites).

Le milieu réactionnel utilisé pour l'amplification des ITR14 et des ITR21 contenait, dans un volume final de 50 µl, la matrice : 10 µl de l'éluat Tnp/ITR ou 10 µl de l'éluat MBP, en présence de 5 µl de tampon 10X; 200 µM de dNTP; 2,5 mM de MgCl₂; 1 µM d'amorces R et F et 5 unités de Taq polymérase. Sur gel d'agarose, l'amplification à partir des éluats Tnp/ITR devait donner une bande de 79 pb et de 300 pb pour l'ITR3' (témoin positif). Les produits PCR ont été purifiés à partir du gel d'agarose, puis marqués en utilisant la PNK et utilisés pour un nouveau tour de SELEX.

Au cinquième tour de SELEX, la PCR a été réalisée en présence de matériel radioactif pour marquage au [α³² P]ATP.

### b) Clonage et séquencage des séquences sélectionnées

Les produits de PCR purifiés du tour numéro 7 de la méthode SELEX 1 et du tour 8 de la méthode SELEX 2 ont été clonés dans le plasmide pGEMT-Easy (kit pGEMT-Easy Vector system, Promega) dans les conditions préconisées par le fournisseur. Les ligatures dans le pGEMT-easy ont été réalisées avec les fragments ITR14 méthode SELEX 1, ITR14 méthode SELEX 2, ITR21 méthode SELEX 1 et ITR21 méthode SELEX 2. Les ligations ont été utilisées pour transformer des bactéries compétentes DH5α. L'ADN plasmidique de 20 clones recombinants de chacune des ligatures a été analysé par séquençage simple brin. Un alignement des séquences a été réalisé grâce au logiciel CLUSTALW accessible sur le site www.infobiogen.fr.

### c) Criblage rapide des séquences clonées

Chacun des ITR potentiels a été testé pour sa capacité à se fixer sur la transposase par retard sur gel. Les ITR ont ainsi été incubés en présence de MBP-Tnp qui doit provoquer un retard de migration si la transposase est fixée sur l'ITR. Dans un premier temps, un criblage rapide des ITR a été réalisé en incubant les ADN radiomarqués par PCR, sans purification, en présence de la protéine. Dans un deuxième temps, un retard sur gel a été effectué afin d'éliminer le bruit de fond dû à l'amplification de séquences artefactuelles.

### c1) Marquage radioactif des séquences clonées

### (i) Marquage par PUR

80 ITR ont été sélectionnés. Ces ITR ont été marqués par PCR, en présence de [α³² P]ATP et des amorces universelles pU et pREV, à partir des minipréparations d'ADN plasmidique.

La taille attendue des fragments amplifiés était de 79 pb.

### (ii) Marquage par remplissage à la Klenow

Les ITR positifs lors du criblage rapide ont été purifiés sur gel d'agarose après digestion par l'enzyme *Eco*RI qui permet d'éliminer les amorces R et F. L'ITR3' a été purifié après digestion du plasmide pBluescript-ITR3'par les enzymes *Eco*RI et *Bam*HI. Ces fragments purifiés ont été radiomarqués à l'aide de [α³² P]ATP par remplissage de site grâce à la Klenow.

### c2) Formation des complexes ADN/protéine

### (i) Criblage rapide

Les complexes ITP/protéine ont été formés avec les séquences marquées par PCR à l'issue du dernier cycle de la réaction SELEX, sans purification préalable, afin de réaliser un criblage rapide. Ces séquences contenaient un ITR encadré par les amorces R et F. La réaction d'interaction contenait, dans un volume final de 20 µl: 40 µg de protéine MBP-Tnp ou MBP; 1 µl de la réaction de PCR radioactive; 1 µg d'ADN de sperme de saumon; 2 µl de glycérol 50 %; 5 mM de MgCl₂ et 0,5 µM de pRev. Les sondes libres ont été préparées avec 1 µl de PCR radioactive, 2 µl de glycérol 50 % et 17 µl de tampon. Les réactions d'interaction ont été maintenues pendant 15 minutes à 4° C avant d'être analysées sur gel de polyacrylamide.

### (ii) Retard sur gel sur les sondes purifiées

Les séquences provoquant un retard de migration après incubation avec la Tnp (ITR positifs) ont été soumises à un nouveau retard sur gel avec un fragment d'ADN purifié. Les complexes ITR/protéine ont été formés dans un volume final de 20 µl contenant: 40 µg de protéine MBP-Tnp, 1 nM de sonde ITR, 1 µg d'ADN de sperme de saumon, 2 µl de glycérol 50 %, 5 mM de MgCl₂ et 0,5 µM de pRev. Les ITR seuls ont été utilisés à une concentration finale de 1 nM dans un mélange contenant 2 µl de glycérol 50 % et 17 µl de tampon. Les réactions d'interaction ont été maintenues pendant 15 minutes à 4° C avant d'être analysées sur gel de polyacrylamide.

### d) Tests de compétition

Le principe de ces tests est illustré à la Figure 9. Ce test permet de mettre en évidence les capacités de l'ITRSelex à déplacer la fixation de la transposase de l'ITR3' radiomarqué. Plus le déplacement est important, plus la séquence ITRSelex sera « améliorée » par rapport à l'ITR3'. En pratique, la réaction de fixation de la transposase est réalisée dans 20 µl contenant 10mM de tampon Tris pH9, 0,5 mM de DDT, 5mM MgCl₂, 5% (vol/vol) de glycérol, 1µg d'ADN de sperme de hareng et 100 ng de BSA, en présence de 15nM d'ITR3' radiomarqué et de l'ITRSelex non radiomarqué. Les concentrations d'ITRSelex non radiomarqué testées étaient de 0nM, 15nM, 75nM, 150nM, 300nM, 750nM, 1500nM.

### e) Construction des plasmides pBC3TSelex

Afin d'analyser le comportement des 8 ITR Selex *in vivo* en bactérie, une série de huit plasmides a été construite à partir du plasmide pBC3T5. Le plasmide pBC3T5 contient l'ORF de *Tet* (gène de résistance à la tétracycline) sans promoteur, bordé par les ITR3' et 5' de *Mos-I.* Le gène *Tet* (cloné entre les sites de restrictions *Xba*1 et *Hind*III) est en orientation inverse du gène de résistance au chloramphénicol et du gène codant pour la protéine LacZ. L'ITR3' est délimité par le site de restriction *Kpn* I du plasmide pBCKS+ en 5' et par le site de restriction *Sal* I en 3'. L'ITR5' est délimité par le site de restriction *Sac*I du plasmide pBCKS+ en 5' et par le site de restriction *Not*I en 3'. L'ITR5' du plasmide pBC3T5 a été remplacé par les ITRSelex après double digestion par *Not*I et *Sac*I, générant les plasmides pBC3TSelex. Les ITRSelex ont été synthétisés sous forme d'oligonucléotides simples brins par la société MWG Biotech (Allemagne). La formation d'ITRSelex double brin a été faite par hybridation de manière à générer des hémi-sites cohésifs *Not*I et *Sac*I. Les oligonucléotides cohésifs ont été désignés de telle sorte qu'un dinucléotide TA bordant l'ITRSelex en 5' soit disposé à l'extérieur du pseudo-élément. Ces oligonucléotides double brin phosphorylés ont été raboutés par l'ADN ligase T4 au vecteur digéré par les deux enzymes, afin de générer les plasmides pBC3TSelex. Ces plasmides sont notés par la suite pBC3Ts ou pBC3TSelex, suivi du numéro de l'ITRSelex.

### f) Tests de transposition

Une description détaillée du protocole expérimental est fournie dans la partie I, paragraphe I-B ci-dessus.

Ces tests ont été réalisés sur des bactéries *E. coli* JM109 cotransformées par 10 ng de plasmide donneur de transposase (pKK-Tnp ou pKK) et 10ng de plasmide donneur de pseudo-transposon (pBC3TSelex). Ces bactéries ont été sélectionnées sur un milieu contenant de l'ampicilline et du chloramphénicol. Les conditions opératoires de ces tests (« Méthode I ») sont indiquées sur la Figure 10.

### I- 2- Résultats

### a) Criblage des séquences d'ITR candidates obtenues par SELEX

La méthode SELEX mise au point par les Inventeurs utilise un mélange d'oligonucléotides de 79 pb formés d'un ITR de 29 pb dégénéré sur 14 ou 21 positions (ITR14 et ITR21), et bordé à ses extrémités par des amorces R et F de 25 pb (SEQ ID N°35 et 36). Elle utilise également une protéine recombinante fusionnant la transposase de *Mos1* (notée Tnp) et la MBP.

Deux méthodes SELEX ont été développées. Le principe général de ces deux méthodes reste identique. Dans la méthode SELEX 1, les oligonucléotides, la protéine et la résine de maltose sont incubés en même temps. Dans la méthode SELEX 2, les matrices sont incubées avec la protéine pendant 5 minutes à 4 °C avant d'être mises en contact avec la résine de maltose.

Les séquences ITR14 et ITR21 sélectionnées au tour 7 de la méthode SELEX 1 et au tour 8 de la méthode SELEX 2 ont été clonées. Pour chaque méthode, 20 clones correspondant aux ITR14 et 20 clones correspondant aux ITR21 ont été isolés puis séquences. Ces 80 séquences ont été analysées en fonction de leur nature, c'est à dire s'il s'agit d'un ITR14 dégénéré sur 14 positions ou d'un ITR21 dégénéré sur 21 positions, et la méthode dont elles sont issues (méthode SELEX 1 ou méthode SELEX 2). Les résultats ont montré que la méthode utilisée pouvait avoir une incidence sur le type de sélection effectuée (données non montrées).

Les 80 séquences ont été testées en retard sur gel pour contrôler leur capacité de liaison avec la transposase. Les résultats ont montré que les clones ITR 1, 6, 9, 40, 46, 49, 60 et 69 (ITRSelex ; Figure 11 ; SEQ ID N°38 à 45) sont capables de former un complexe avec la protéine. Ces clones positifs ont été testés en retard sur gel afin de savoir s'ils sont reconnus par la transposase comme des ITR ou comme des cibles. Les résultats ont montré que les clones 1, 40, 46, 49 et 69 sont des ITR (Figure 11 et données non montrées). Les résultats n'ont pas permis de conclure pour les clones 6 et 9 (Figure 11).

### b) Tests de compétition

Les résultats ont montré que, sur les 8 ITR retenus d'après les expériences de retard sur gel ci-dessus, seuls les ITR40 et 46 sont capables d'inhiber la fixation de la transposase sur l'ITR3' radiomarqué.
ITR40 5'-TCAGGTGTACAAGTATGTAATGTCGTTA-3' (SEQ ID N°39);
ITR46 5'- TCAGGTGTACAAGTATGAGATGTCGTTT-3' (SEQ ID N°38).

Comme l'illustre la Figure 12, seuls les ITR40 et 46 sont capables d'entrer en compétition avec l'ITR3' et de déplacer la fixation de la transposase de l'ITR3' radiomarqué.

### c) Tests de transposition

Bien que les ITR40 et 46 apparaissent comme les meilleurs candidats d'après les tests de compétition, le comportement des 8 ITR a été évalué en test de transposition *in vivo* en bactérie, afin de vérifier si et dans quelle mesure ces ITR ont la capacité de médier l'ensemble de la transposition.

Les résultats obtenus sont présentés sur la Figure 13 A et B. Il apparaît que seuls les ITR40 et 46 permettent effectivement d'améliorer la transposition dans les conditions testées. S'agissant des témoins (ou contrôles), dans les conditions expérimentales de la méthode I, l'efficacité de la transposition de pBC3T3 était augmentée d'un facteur 10 par rapport à celle obtenue avec le plasmide pBC3T5 (Fig. 13A).

Finalement, comme le montre la Fig. 13B, les pseudo-transposons 3T40 et 3T46 sont hyperactifs.

### II- Les UTR (Untranslated Terminal Repeat)

La configuration minimale des acides nucléiques pour une transposition optimale de l'élément *Mos-1* semble inclure, outre les ITR, une partie au moins des UTR. En effet, *in vitro*, un marqueur de résistance uniquement bordé par les ITR 5' et 3' de *Mos-1* ne transpose pas, alors que l'ajout des 38 premières pb de l'UTR 5' et des 5 premières pb de l'UTR 3' aux séquences des ITR respectifs suffit à rétablir l'activité sauvage (Tosi et al., 2000).

La nécessité de la présence des UTR à proximité des ITR a été évaluée par construction d'un certain nombre de configurations possibles : UTR en 5' ou 3', UTR 5' associé à un ITR 3', ou inversement.

En bref, les résultats obtenus ont montré que la présence d'UTR favorise la transposition (notamment, amélioration d'un facteur au moins égal à environ 5).

### II- 1- Matériel et méthodes

### a) Construction des configurations ITR+UTR

### a1) Plasmides

Les plasmides ITR/UTR ont tous été construits à partir du plasmide pBC3T5 selon le même mode opératoire. L'ITR3' a été remplacé après double digestion par les enzymes *Kpn*I et *Sal*I. L'ITR5' a été remplacé par double digestion *Not*I et *Sac*I. Les différentes séquences ITR/UTR 33 et 55 ont été synthétisées et clonées dans pCR4-TOPO (Invitrogen) par la société ATG biosynthetics (Allemagne). La séquence ITR/UTR35 - MCS - UTR/ITR35, c'est-à-dire ITR3'/UTR5' - Site de clonage multiple (MCS: « Multiple Cloning Site ») - UTR3'/ITR5' a été synthétisée et clonée dans pCR-Script AmpSK(+) (Stratagene) par la société Intelechon (Allemagne). Cette séquence a été introduite dans pBC par double digestion *Kpn*I et *Sac*I. Ces séquences ont été désignées de telle sorte qu'un dinucléotide TA bordant l'ITR/UTR en 5' soit disposé à l'extérieur du pseudo-élément. Une quinzaine de constructions ont été réalisées et évaluées dans les tests de transposition en bactérie selon la méthode 2. Les résultats sont présentés pour les plasmides suivants avec la notation pBC ITR/UTR-T-UTR/ITR: pBC33T33, pBC33T55 et pBC35T35.

Le plasmide donneur de transposase pKKTnp est un dérivé du plasmide pKK233-2 (Clontech ; Amp^{r}) dans lequel a été cloné, au site *Nco*I, l'ORF de la transposase *Mos-l* noté Tnp. Son expression est sous le contrôle du promoteur inductible à l'IPTG *Ptrc* (ce promoteur possède cependant une activité transcriptionnelle de base en l'absence d'inducteur ; données non montrées). Ce plasmide pKK233-2 sera simplement noté pKK par la suite lorsque l'ORF de la transposase est absent.

### a2) Transformation des souches E. coli JM109 pour tests de transposition

Les bactéries JM109 compétentes ont été co-transformées avec un plasmide donneur de transposon dont les plasmides pBC33T33, pBC3T5, pBC3T3, pBC33T55, pBC35T35, pBC3T33, et le plasmide donneur de Tnp pKKTnp. Des souches témoins ont été co-transformées avec les mêmes plasmides donneurs de transposon et le plasmide témoin pKK.

### b) Tests de transposition

Une description détaillée du protocole expérimental est fournie dans la partie I, paragraphe I-B ci-dessus.

La quinzaine de configurations a été testée en transposition *in vivo* en bactéries selon la méthode II, illustrée par la Figure 14.

### II- 2- Résultats

L'efficacité de transposition a été calculée en divisant le nombre de clones Tet^{R} apparus par le nombre de bactéries analysées en présence du plasmide donneur de transposase pKK-Tnp, auquel il a été ôté le bruit de fond de l'expérience obtenu en présence du plasmide témoin pKK. Les résultats les plus significatifs ont été obtenus pour les constructions 3T33, 33T33 et 35T35, en comparaison avec les constructions contrôles 3T3, 3T5 et 33T55. L'efficacité de la transposition était augmentée d'un facteur 5 et 20 pour les constructions pBC33T33 et pBC3T33, par rapport à la construction témoin pBC3T3. Ces résultats montrent que la présence de la séquence UTR est extrêmement importante pour la réaction de transposition puisque l'on observe une efficacité de transposition, avec la construction pBC33T33, 300 fois supérieure à celle obtenue pour le plasmide pBC3T5 comme pour le plasmide pBC33T55. Les meilleurs résultats ont été obtenus pour la construction pBC35T35, qui donne une augmentation de l'efficacité de la transposition d'un facteur 54000 par rapport à pBC3T5 et d'un facteur 1000 par rapport à pBC3T3.

Selon la Figure 15A, les constructions intéressantes sont pBC35T35, pBC3T33 et pBC33T33.

La Figure 15B montre que les pseudo-transposons 3T33, 33T33 et 35T35 sont hyperactifs.

Les Inventeurs ont en outre testé les constructions 53T35, 53T33, 35T33, 55T35, 53T55, 55T55, 5T35, 5T33, 3T55, 3T53. Ils ont ainsi pu observer que ces constructions n'entraînaient pas d'hyperactivité ; elles étaient d'efficacité soit équivalente à celle du 3T5 ou du 3T3, soit plus faible ou nulle (données non montrées).

### PARTIE III: SYSTEMES DE TRANSPOSITION RECOMBINANTS HYPERACTIFS COMPRENANT UNE TRANSPOSASE MOS-1 MUTANTE HYPERACTIVE (Tnp) ET UN PSEUDO-TRANSPOSON Mos-1 RECOMBINANT HYPERACTIF

Pour évaluer l'efficacité de transposition de systèmes associant une transposase hyperactive et un pseudo-transposon également hyperactif, diverses combinaisons ont été testées dans le test de transposition en bactérie décrit dans la partie I, paragraphe I-B ci-dessus.

Dans ce test, le plasmide pBC3T3 a été remplacé par les pseudo-transposons hyperactifs pBC3T33, pBC3T40, pBC3T46. Le plasmide pKK-Tnp sauvage a été remplacé par des vecteurs de type pKK exprimant chacun une transposase mutante hyperactive particulière.

Le Tableau 3 ci-dessous donne les résultats obtenus en combinant des transposases hyperactives (FETY, FQETY, FTY, FT, TY, ET, FQ, FQET, QY) et des pseudo-transposons 3T33, 3T40, 3T46, 33T55 (ce dernier étant utilisé comme contrôle).

**Tableau 3**

| | | Facteur d'amplification par rapport à 3T3/WT | | | | Facteur d'amplification par rapport à 3T3/WT | |
|---|---|---|---|---|---|---|---|
| | | T0 | T5 | | | T0 | T5 |
| 3T40/ | | | | 33T55/ | | | |
| | FETY | 4 | 0,1 | | FETY | 1,1 | 1,5 |
| | FQETY | 2,5 | 0,8 | | FQETY | 0,4 | 1,6 |
| | FTY | 3 | 2.4 | | FTY | 2,1 | 4,6 |
| | FT | 0 | 2 | | FT | 0 | 0,1 |
| | TY | 1,4 | 36,2 | | TY | 0 | 8,4 |
| | ET | 4,1 | 8,9 | | ET | 0 | 7,1 |
| | FQ | 0,7 | 2,5 | | FQ | 0 | 0,1 |
| | FQET | 1,8 | 3,7 | | FQET | 0 | 0.2 |
| | QY | 0,3 | 1,3 | | QY | 0 | 0 |
| | | | | | | | |
| 3T46/ | | | | 3T33/ | | | |
| | FETY | 6,5 | 2 | | FETY | 24,6 | 51.4 |
| | FQETY | 3,6 | 2,3 | | FQETY | 22,4 | 59,7 |
| | FTY | 6,7 | 19,4 | | FTY | 44 | 56 |
| | FT | 0,1 | 3,7 | | FT | 42,9 | 60 |
| | TY | 2,3 | 61,8 | | TY | 68,4 | 66,5 |
| | ET | 15,6 | 6,7 | | ET | 37,6 | 218,5 |
| | FQ | 1,4 | 3,3 | | FQ | 4,3 | 109,3 |
| | FQET | 3,3 | 12,3 | | FQET | 36 | 63,5 |
| | QY | 1,7 | 1,7 | | QY | 5,2 | 28 |

| | | | | | | | |
|---|---|---|---|---|---|---|---|
| WT : transposase Mos-1 sauvage F : transposase Mos-1 avec la mutation F53Y E : transposase Mos-1 avec la mutation E137K T : transposase Mos-1 avec la mutation T216A Y : transposase Mos-1 avec la mutation Y237C Q : transposase Mos-1 avec la mutation Q91R | | | | | | | |

De manière tout à fait surprenante et imprévisible, les résultats obtenus sur les combinaisons testées révèlent que toutes les combinaisons d'une transposase Mos-1 hyperactive avec un pseudo-transposon *Mos-1* hyperactif ne sont pas nécessairement hyperactives.

Les résultats ainsi obtenus permettent de sélectionner, en tant que combinaisons intéressantes aux fins de la présente invention, les associations :
- pseudo-transposon 3T40 + transposase TY,
- pseudo-transposon 3T46 + transposase TY ou ET ou FTY
- pseudo-transposon 3T33 + transposase TY ou ET ou FQ ou FQET.

Dans les conditions des tests de transposition en bactérie ici rapportés, la combinaison pseudo-transposon 3T33 + transposase ET est la plus intéressante puisqu'elle est respectivement plus efficace que l'association pseudo-transposon 3T3 + transposase WT (200 fois), pseudo-transposon 3T3 + transposase FETY (3,5 fois) et pseudo-transposon 3T33 + transposase WT (10 fois).

Une analyse biochimique en gel retard a été effectuée en suivant les procédures décrites précédemment [notamment, dans la demande internationale WO 2004/078981 publiée le 16 septembre 2004 et dans Augé-Gouillou et al. (2001 b)] afin de déterminer la stabilité des complexes transposase + ITR ou transposase + ITR / UTR.

Ces travaux ont permis de montrer que les fragments d'ADN associant l'ITR3' à l'UTR3' et l'ITR3' à l'UTR5' sont beaucoup plus stables (4 fois) que ceux formés avec les ITRs seuls ou d'autres combinaisons (Fig. 16). Cette plus grande stabilité des complexes pourrait être à l'origine de l'hyperactivité observée.

### REFERENCES

Lampe DJ. et al. (1996) EMBO J. 15 : 5470-5479
Plasterk RHA. et al. (1999) Trends in genetics 15 : 326-332
Renault S. et al. (1997) Virologie 1 : 133-144
Jacobson et Hartl (1985) Genetics 111 : 57-65
Cralg et al. (2002) Mobile DNA II. ASM Press. Washington. USA
Jeong et al. (2002) PNAS 99 : 1076-1081
Martienssen et Colot (2001) Science 293 : 1070-1074
Ketting et al. (1999) Cell 99 : 1.33-141
Tabara et al. (1999) Cell 99 : 123-132
Ausubel et al. (1994) In Janssen, K. (Ed) Current Protocols in Molecular Biology. J. Wiley & Sons, Inc. Massachussetts General Hospital. Harvard Medical School
Augé-Gouillou et al. (2001) Mol. Genet. Genomics 265 : 58-65
Augé-Gouillou et al. (2001b) Mol. Genet. Genomics 265 : 51-57
Turner DL et al. (1994) Gènes Dev. 8 : 1434-1447
Sambrook et Russel (2001) Molecular Cloning: a laboratory manual (3rd Ed.) Cold Spring Harbor Laboratory Press, Cold Spring Harbor, NY.
Tosi et al. (2000) Nucleic Acids Res. 28: 784-790
Ellington et al. (1990) Nature 346 : 818-822
Tuerk et al. (1990) Science 249 : 505-510
Klug et al. (1994) Mol. Biol. Rep. 20 : 97-107
Zhang et al. (2001) Nucleic Acids Res. 29 :3566-3575

## Revendications

1. Pseudo-transposon *Mos-1* hyperactif, dans lequel :
a) au moins l'une des deux répétitions terminales non traduites (UTR) et/ou au moins l'une des deux répétitions terminales inversées (ITR) est(sont) génétiquement modifiée(s) ; et
b) une séquence nucléotidique exogène d'intérêt remplace la séquence nucléotidique codant la transposase Mos-1 d'origine,
**caractérisé en ce qu'**il est choisi parmi les pseudo-transposons suivants :
α) ITR3'-UTR3'-séquence nucléotidique exogène d'intérêt-UTR3'-ITR3' (pseudo-transposon 33seq33),
β) ITR3'-séquence nucléotidique exogène d'intérêt-UTR3'-ITR3' (pseudo-transposon 3seq33),
γ) ITR3'-UTR5'-séquence nucléotidique exogène d'intérêt-UTR3'-ITR5' (pseudo-transposon 35seq35),
δ) les pseudo-transposons comprenant au moins une ITR40 de séquence SEQ ID n°39, et
ε) les pseudo-transposons comprenant au moins une ITR46 de séquence SEQ ID n°38, et dans lequel ITR3', UTR3', UTR5' et ITR5' désignent respectivement la séquence de la répétition terminale inversée 3' du transposon Mos-1, la séquence de la répétition terminale non-traduite 3' du transposon Mos-1, la séquence de la répétition terminale non-traduite 5' du transposon Mos-1 et la séquence de la répétition terminale inversée 5' du transposon Mos-1.

2. Système de transposition recombinant hyperactif dérivé du transposon *Mos-1,* comprenant au moins les deux partenaires suivants :
a) un pseudo-transposon *Mos-1* hyperactif selon la revendication 1 ; et
b) une transposase Mos-1 fournie en *trans* dudit pseudo-transposon, dans lequel la fréquence de transposition de ladite séquence nucléotidique exogène d'intérêt est améliorée d'un facteur au moins égal à 5, de préférence au moins égal à 10, par rapport à la fréquence de transposition de la-dite séquence nucléotidique exogène d'intérêt portée par un pseudo-transposon Mos-1 bordé de deux ITR3' (3seq3) en présence de la transposase Mos-1 fournie en *trans*.

3. Système selon la revendication 2, **caractérisé en ce que** ladite transposase Mos-1 fournie en *trans* est une transposase mutante.

4. Système selon la revendication 3, **caractérisé en ce que** ladite transposase Mos-1 mutante est hyperactive.

5. Système selon la revendication 4, **caractérisé en ce que** ladite transposase Mos-1 mutante hyperactive comprend au moins une mutation au niveau d'au moins un résidu choisi parmi les résidus suivants de la séquence SEQ ID N°2 : F53, Q91, E137, T216 et Y237.

6. Système selon la revendication 5, **caractérisé en ce qu'**il comprend au moins les deux partenaires suivants :
a) un pseudo-transposon *Mos-1* hyperactif comprenant au moins une ITR40 de séquence SEQ ID n°39 et une transposase Mos-1 mutante hyperactive comprenant les mutations T216A et Y237C ;
b) un pseudo-transposon *Mos-1* hyperactif comprenant au moins une ITR46 de séquence SEQ ID n°38 et une transposase Mos-1 mutante hyperactive comprenant les mutations T216A et Y237C, ou E137K et T216A, ou F53Y et T216A et Y237C ;
c) un pseudo-transposon *Mos-1* hyperactif 3seq33 et une transposase Mos-1 mutante hyperactive comprenant les mutations T216A et Y237C, ou E137K et T216A, ou F53Y et Q91R, ou F53Y et Q91R et E137K et T216A.

7. Pseudo-transposon selon la revendication 1 ou système selon l'une quelconque des revendications 2 à 6, **caractérisé en ce que** ladite séquence nucléotidique exogène d'intérêt est un gène fonctionnel.

8. Vecteur comprenant au moins un pseudo-transposon selon la revendication 1.

9. Cellule hôte comprenant au moins :
a) un pseudo-transposon selon la revendication 1 ou 7 ; ou
b) un système selon l'une quelconque des revendications 2 à 7 ; ou
c) un vecteur selon la revendication 8 ; ou
d) une combinaison de ceux-ci.

10. Kit comprenant au moins :
a) un pseudo-transposon selon la revendication 1 ou 7 ; ou
b) un système selon l'une quelconque des revendications 2 à 7 ; ou
c) un vecteur selon la revendication 8 ; ou
d) une cellule hôte selon la revendication 9 ; ou
e) une combinaison de ceux-ci.

11. Utilisation d'au moins :
a) un pseudo-transposon selon la revendication 1 ou 7 ; ou
b) un système selon l'une quelconque des revendications 2 à 7 ; ou
c) un vecteur selon la revendication 8 ; ou
d) une cellule hôte selon la revendication 9 ; ou
e) un kit selon la revendication 10 ; ou
f) une combinaison de ceux-ci,
pour la transposition efficace *in vitro* ou *ex vivo* d'une séquence nucléotidique exogène d'intérêt.

12. Utilisation d'au moins :
a) un pseudo-transposon selon la revendication 1 ou 7 ; ou
b) un système selon l'une quelconque des revendications 2 à 7 ; ou
c) un vecteur selon la revendication 8 ; ou
d) une cellule hôte selon la revendication 9 ; ou
e) un kit selon la revendication 10 ; ou
f) une combinaison de ceux-ci,
pour la mutagénèse insertionnelle *in vitro* ou *ex vivo.*

13. Utilisation d'au moins :
a) un pseudo-transposon selon la revendication 1 ou 7 ; ou
b) un système selon l'une quelconque des revendications 2 à 7 ; ou
c) un vecteur selon la revendication 8 ; ou
d) une cellule hôte selon la revendication 9 ; ou
e) un kit selon la revendication 10 ; ou
f) une combinaison de ceux-ci,
pour le séquençage et/ou le clonage d'acides nucléiques.

14. Procédé de préparation d'un médicament, comprenant au moins une étape de transposition *in vitro* ou *ex vivo* d'une séquence d'ADN transposable d'intérêt dans une séquence d'ADN cible, ladite transposition étant médiée par au moins l'un des moyens suivants :
a) un pseudo-transposon selon la revendication 1 ou 7 ; ou
b) un système selon l'une quelconque des revendications 2 à 7 ; ou
c) un vecteur selon la revendication 8 ; ou
d) une cellule hôte selon la revendication 9 ; ou
e) un kit selon la revendication 10 ; ou
f) une combinaison de ceux-ci.

15. Utilisation d'au moins :
a) un pseudo-transposon selon la revendication 1 ou 7 ; ou
b) un système selon l'une quelconque des revendications 2 à 7 ; ou
c) un vecteur selon la revendication 8 ; ou
d) une cellule hôte selon la revendication 9 ; ou
e) un kit selon la revendication 10 ; ou
f) une combinaison de ceux-ci,
pour la préparation d'un médicament destiné à permettre la transposition efficace *in vivo* d'une séquence nucléotidique exogène d'intérêt.

## Claims

1. Hyperactive Mos-1 pseudo-transposon, in which:
a) at least one of the two untranslated terminal repeats (UTR) and/or at least one of the two inverted terminal repeats (ITR) is/are genetically modified; and
b) an exogenous nucleotide sequence of interest replaces the nucleotide sequence encoding the original Mos-1 transposase;
**characterised in that** it is selected from the following pseudo-transposons:
α) ITR3'-UTR3'-exogenous nucleotide sequence of interest-UTR3'-ITR3' (pseudo-transposon 33seq33),
p) ITR3'-exogenous nucleotide sequence of interest-UTR3'-ITR3' (pseudo-transposon 3seq33),
γ) ITR3'-UTR5'-exogenous nucleotide sequence of interest-UTR3'-ITR3' (pseudo-transposon 35seq35),
δ) the pseudo-transposons comprising at least one ITR40 having the sequence SEQ ID No. 39, and
ε) the pseudo-transposons comprising at least one ITR46 having the sequence SEQ ID No. 38,
and in which ITR3', UTR3', UTR5' and ITR5' refer respectively to the 3' inverted terminal repeat sequence of Mos-1 transposon, the 3' untranslated terminal repeat sequence of Mos-1 transposon, the 5' untranslated terminal repeat sequence of Mos-1 transposon and the 5' inverted terminal repeat sequence of Mos-1 transposon.

2. A hyperactive recombinant transposing system derived from Mos-1 transposon, comprising at least the two following partners:
a) a hyperactive Mos-1 pseudo-transposon according to claim 1; and
b) a Mos-1 transposase provided in trans to said pseudo-transposon,
wherein the transposition frequency of said exogenous nucleotide sequence of interest is improved by a factor at least equal to 5, preferentially at least equal to 10, compared to the transposition frequency of said exogenous nucleotide sequence of interest borne by a Mos-1 pseudo-transposon flanked by two ITR3' (3seq3) in the presence of Mos-1 transposase provided in trans.

3. System according to claim 2 **characterised in that** said Mos-1 transposase provided in trans is a mutant transposase.

4. System according to claim 3, **characterised in that** said mutant Mos-1 transposase is hyperactive.

5. System according to claim 4, **characterised in that** the hyperactive mutant Mos-1 transposase comprises at least one mutation on at least one residue selected from the following residues of the sequence SEQ ID No. 2: F53, Q91, E137, T216 and Y237.

6. System according to claim 5, **characterised in that** it comprises at least the two following partners:
a) a hyperactive Mos-1 pseudo-transposon comprising at least one ITR40 having the sequence SEQ ID No. 39 and a hyperactive mutant Mos-1 transposase comprising the mutations T216A and Y237C;
b) a hyperactive Mos-1 pseudo-transposon comprising at least one ITR46 having the sequence SEQ ID No. 38 and a hyperactive mutant Mos-1 transposase comprising the mutations T216A and Y237C, or E137K and T216A, or F53Y and T216A and Y237C;
c) A hyperactive Mos-1 pseudo-transposon 3seq33 and a hyperactive mutant Mos-1 transposase comprising the mutations T216A and Y237C, or E137K and T216A, or F53Y and Q91R, or F53Y and Q91R and E137K and T216A.

7. Pseudo-transposon according to claim 1 or system according to any of claims 2 to 6, **characterised in that** said exogenous nucleotide sequence of interest is a functional gene.

8. Vector comprising at least one pseudo-transposon according to claim 1.

9. Host cell comprising at least:
a) one pseudo-transposon according to claim 1 or 7; or
b) one system according to any of claims 2 to 7; or
c) one vector according to claim 8; or
d) a combination thereof.

10. Kit comprising at least:
a) one pseudo-transposon according to claim 1 or 7; or
b) one system according to any of claims 2 to 7; or
c) one vector according to claim 8; or
d) one host cell according to claim 9; or
e) a combination thereof.

11. Use of at least:
a) one pseudo-transposon according to claim 1 or 7; or
b) one system according to any of claims 2 to 7; or
c) one vector according to claim 8; or
d) one host cell according to claim 9; or
e) one kit according to claim 10; or
f) a combination thereof,
for the efficient in vitro or in vivo or ex vivo transposition of an exogenous nucleotide sequence of interest.

12. Use of at least:
a) one pseudo-transposon according to claim 1 or 7; or
b) one system according to any of claims 2 to 7; or
c) one vector according to claim 8; or
d) one host cell according to claim 9; or
e) one kit according to claim 10; or
f) a combination thereof,
for in vitro or ex vivo insertional mutagenesis.

13. Use of at least:
a) one pseudo-transposon according to claim 1 or 7; or
b) one system according to any of claims 2 to 7; or
c) one vector according to claim 8; or
d) one host cell according to claim 9; or
e) one kit according to claim 10; or
f) a combination thereof,
for sequencing and/or cloning nucleic acids.

14. A method for preparing a medicinal product, comprising at least one in vitro or ex vivo transposition step of a transposable DNA sequence of interest in a target DNA sequence, said transposition being mediated by at least one of the following means:
a) one pseudo-transposon according to claim 1 or 7; or
b) one system according to any of claims 2 to 7; or
c) one vector according to claim 8; or
d) one host cell according to claim 9; or
e) one kit according to claim 10; or
f) a combination thereof.

15. Use of at least:
a) one pseudo-transposon according to claim 1 or 7; or
b) one system according to any of claims 2 to 7; or
c) one vector according to claim 8; or
d) one host cell according to claim 9; or
e) one kit according to claim 10; or
f) a combination thereof,
for preparing a medicinal product for enabling the effective in vivo transposition of an exogenous nucleotide sequence of interest.

## Patentansprüche

1. Hyperaktives Pseudo-Transposon *Mos-1*, in welchem:
a) mindestens eine der beiden nicht-translatierten terminalen Sequenzwiederholungen (UTR) und/oder mindestens eine der beiden invertierten terminalen Sequenzwiederholungen (ITR) genetisch modifiziert ist/sind; und
b) eine exogene Nukleotidsequenz von Interesse die Nukleotidsequenz, die die ursprüngliche Mos-1-Transposase kodiert, ersetzt,
**dadurch gekennzeichnet, dass** es ausgewählt ist unter den folgenden Pseudo-Transposons:
α) ITR3'-UTR3'-exogene Nukleotidsequenz von Interesse-UTR3'-ITR3' (Pseudo-Transposon 33seq33)
β) ITR3'-exogene Nukleotidsequenz von Interesse-UTR3'-ITR3' (Pseudo-Transposon 3seq33)
γ) ITR3'-UTR5'-exogene Nukleotidsequenz von Interesse-UTR3'-ITR5' (Pseudo-Transposon 35seq35)
δ) den Pseudo-Transposons, die mindestens eine ITR40 der Sequenz SEQ ID Nr. 39 umfassen, und
ε) den Pseudo-Transposons, die mindestens eine ITR46 der Sequenz SEQ ID Nr. 38 umfassen,
und in welchem ITR3', UTR3', UTR5' und ITR5' jeweils die Sequenz der invertierten terminalen 3'-Sequenzwiederholung des Transposons Mos-1,
die Sequenz der nicht-translatierten terminalen 3'-Sequenzwiederholung des Transposons Mos-1, die Sequenz der nicht-translatierten terminalen 5'-Sequenzwiederholung des Transposons Mos-1 bzw. die Sequenz der invertierten terminalen 5'-Sequenzwiederholung des Transposons Mos-1 bezeichnen.

2. Hyperaktives rekombinantes Transpositionssystem, welches von dem Transposon *Mos-1* abgeleitet ist, welches mindestens die folgenden zwei Partner umfasst:
a) ein hyperaktives Pseudo-Transposon *Mos-1* gemäß Anspruch 1 und
b) eine Mos-1-Transposase, die in *trans* zu dem Pseudo-Transposon bereitgestellt wird,
in welchem die Transpositionsfrequenz der exogenen Nukleotidsequenz von Interesse um einen Faktor von mindestens gleich 5, vorzugsweise mindestens gleich 10 verbessert ist bezogen auf die Transpositionsfrequenz der exogenen Nukleotidsequenz von Interesse, die sich innerhalb eines Pseudo-Transposons Mos-1, eingerahmt von zwei ITR3' (3seq3), befindet, in Gegenwart der in *trans* bereitgestellten Mos-1-Transposase.

3. System nach Anspruch 2, **dadurch gekennzeichnet, dass** die in *trans* bereitgestellte Mos-1-Transposase eine mutierte Transposase ist.

4. System nach Anspruch 3, **dadurch gekennzeichnet, dass** die mutierte Mos-1-Transposase hyperaktiv ist.

5. System nach Anspruch 4, **dadurch gekennzeichnet, dass** die mutierte hyperaktive Mos-1-Transposase mindestens eine Mutation auf der Ebene von mindestens einem Rest, der unter den folgenden Resten der Sequenz SEQ ID Nr. 2 ausgewählt ist: F53, Q91, E137, T216 und Y237, umfasst.

6. System nach Anspruch 5, **dadurch gekennzeichnet, dass** es mindestens die beiden folgenden Partner umfasst:
a) ein hyperaktives Pseudo-Transposon *Mos-1,* umfassend mindestens eine ITR40 der Sequenz SEQ ID Nr. 39 und eine hyperaktive mutierte Mos-1-Transposase, welche die Mutationen T216A und Y237C umfasst;
b) ein hyperaktives Pseudo-Transposon *Mos-1,* umfassend mindestens eine ITR46 der Sequenz SEQ ID Nr. 38 und eine hyperaktive mutierte Mos-1-Transposase, welche die Mutationen T216A und Y237C oder E137K und T216A oder F53Y und T216A und Y237C umfasst;
c) ein hyperaktives Pseudo-Transposon *Mos-1* 3seq33 und eine mutierte hyperaktive Mos-1-Transposase, welche die Mutationen T216A und Y237C oder E137K und T216A oder F53Y und Q91 R oder F53Y und Q91R und E137K und T216A umfasst.

7. Pseudo-Transposon nach Anspruch 1 oder System nach einem der Ansprüche 2 bis 6, **dadurch gekennzeichnet, dass** die exogene Nukleotidsequenz von Interesse ein funktionelles Gen ist.

8. Vektor, welcher mindestens ein Pseudo-Transposon nach Anspruch 1 umfasst.

9. Wirtszelle, welche mindestens umfasst:
a) ein Pseudo-Transposon nach Anspruch 1 oder 7; oder
b) ein System nach einem der Ansprüche 2 bis 7; oder
c) einen Vektor nach Anspruch 8; oder
d) eine Kombination von jenen.

10. Kit, welcher mindestens umfasst:
a) ein Pseudo-Transposon nach Anspruch 1 oder 7; oder
b) ein System nach einem der Ansprüche 2 bis 7; oder
c) einen Vektor nach Anspruch 8; oder
d) eine Wirtszelle nach Anspruch 9; oder
e) eine Kombination von jenen.

11. Verwendung von mindestens:
a) einem Pseudo-Transposon nach Anspruch 1 oder 7; oder
b) einem System nach einem der Ansprüche 2 bis 7; oder
c) einem Vektor nach Anspruch 8; oder
d) einer Wirtszelle nach Anspruch 9; oder
e) einem Kit nach Anspruch 10; oder
f) einer Kombination von jenen
für die wirksame *in vitro-* oder *ex vivo*-Transposition einer exogenen Nukleotidsequenz von Interesse.

12. Verwendung von mindestens:
a) einem Pseudo-Transposon nach Anspruch 1 oder 7; oder
b) einem System nach einem der Ansprüche 2 bis 7; oder
c) einem Vektor nach Anspruch 8; oder
d) einer Wirtszelle nach Anspruch 9; oder
e) einem Kit nach Anspruch 10; oder
f) einer Kombination von jenen
für die *in vitro-* oder *ex vivo*-Insertionsmutagenese.

13. Verwendung von mindestens:
a) einem Pseudo-Transposon nach Anspruch 1 oder 7; oder
b) einem System nach einem der Ansprüche 2 bis 7; oder
c) einem Vektor nach Anspruch 8; oder
d) einer Wirtszelle nach Anspruch 9; oder
e) einem Kit nach Anspruch 10; oder
f) einer Kombination von jenen
für die Sequenzierung und/oder Klonierung von Nukleinsäuren.

14. Verfahren zur Herstellung eines Arzneimittels, welches mindestens einen Schritt einer *in vitro-* oder *ex vivo*-Transposition einer transponierbaren DNA-Sequenz von Interesse in eine DNA-Zielsequenz umfasst, wobei die Transposition durch mindestens eines der folgenden Mittel vermittelt wird:
a) ein Pseudo-Transposon nach Anspruch 1 oder 7; oder
b) ein System nach einem der Ansprüche 2 bis 7; oder
c) einen Vektor nach Anspruch 8; oder
d) eine Wirtszelle nach Anspruch 9; oder
e) einen Kit nach Anspruch 10; oder
f) eine Kombination von jenen.

15. Verwendung von mindestens:
a) einem Pseudo-Transposon nach Anspruch 1 oder 7; oder
b) einem System nach einem der Ansprüche 2 bis 7; oder
c) einem Vektor nach Anspruch 8; oder
d) einer Wirtszelle nach Anspruch 9; oder
e) einem Kit nach Anspruch 10; oder
f) einer Kombination von jenen
für die Herstellung eines Arzneimittels, welches dazu bestimmt ist, die wirksame *in vivo*-Transposition einer exogenen Nukleotidsequenz von Interesse zu erlauben.


### LISTE DE SEQUENCE

<110> Centre National de la Recherche Scientifique (CNRS) Université François Rabelais de Tours
<120> SYSTEMES DE TRANSPOSITION RECOMBINANTS HYPERACTIFS DERIVES DU TRANSPOSON Mos-1
<130> 351306 - D23688
<150> FR0604285
   <151> 2006-05-15
<160> 45
<170> PatentIn version 3.3
<210> 1
   <211> 1038
   <212> DNA
   <213> drosophila mauritiana
<220>
   <223> Séquence du gène codant la transposase du transposon Mos-1
<400> 1
   atgtcgagtt tcgtgccgaa taaagagcaa acgcggacag tattaatttt ctgttttcat     60
   ttgaagaaaa cagctgcgga atcgcaccga atgcttgttg aagcctttgg cgaacaagta    120
   ccaactgtga aaacgtgtga acggtggttt caacgcttca aaagtggtga ttttgacgtc    180
   gacgacaaag agcacggaaa accgccaaaa aggtacgaag acgccgaact gcaagcatta    240
   ttggatgaag acgatgctca aacgcaaaaa caactcgcag agcagttgga agtaagtcaa    300
   caagcagttt ccaatcgctt gcgagagatg ggaaagattc agaaggtcgg tagatgggtg    360
   ccacatgagt tgaacgagag gcagatggag aggcgcaaaa acacatgcga aattttgctt    420
   tcacgataca aaaggaagtc gtttttgcat cgtatcgtta ctggcgatga aaaatggatc    480
   ttttttgtta atcctaaacg taaaaagtca tacgttgatc ctggacaacc gtccacatcg    540
   actgctcgac cgaatcgctt tggcaagaag acgatgctct gtgtttggtg ggatcagagc    600
   ggtgtcattt actatgagct cttgaaaccc ggcgaaacgg tgaatacggc acgctaccaa    660
   caacaattga tcaatttgaa ccgtgcgctt cagagaaaac gaccggaata tcaaaaaaga    720
   caacacaggg tcatttttct ccatgacaac gctccatcac atacggcaag agcggttcgc    780
   gacacgttgg aaacactcaa ttgggaagtg cttccgcatg cggcttactc accagacctg    840
   gccccatccg attaccacct attcgcttcg atgggacacg cactcgctga gcagcgcttc    900
   gattcttacg aaagtgtgaa aaaatggctc gatgaatggt tcgccgcaaa agacgatgag    960
   ttctactggc gtggaatcca caaattgccc gagagatggg aaaaatgtgt agctagcgac   1020
   ggcaaatact ttgaataa                                                 1038
<210> 2
   <211> 345
   <212> PRT
   <213> drosophila mauritiana
<400> 2
   Met Ser Ser Phe Val Pro Asn Lys Glu Gln Thr Arg Thr Val Leu Ile
   1               5                   10                  15
   Phe Cys Phe His Leu Lys Lys Thr Ala Ala Glu Ser His Arg Met Leu
               20                  25                  30
   Val Glu Ala Phe Gly Glu Gln Val Pro Thr Val Lys Lys Cys Glu Arg
           35                  40                  45
   Trp Phe Gln Arg Phe Lys Ser Gly Asp Phe Asp Val Asp Asp Lys Glu
   50 55 60
   His Gly Lys Pro Pro Lys Arg Tyr Glu Asp Ala Glu Leu Gln Ala Leu
   65 70 75 80
   Leu Asp Glu Asp Asp Ala Gln Thr Gln Lys Gln Leu Ala Glu Gln Leu
   85 90 95
   Glu Val Ser Gln Gln Ala Val Ser Asn Arg Leu Arg Glu Met Gly Lys
   100 105 110
   Ile Gln Lys Val Gly Arg Trp Val Pro His Glu Leu Asn Glu Arg Gln
   115 120 125
   Met Glu Arg Arg Lys Asn Thr Cys Glu Ile Leu Leu Ser Arg Tyr Lys
   130 135 140
   Arg Lys Ser Phe Leu His Arg Ile Val Thr Gly Asp Glu Lys Trp Ile
   145 150 155 160
   Phe Phe Val Ser Pro Lys Arg Lys Lys Ser Tyr Val Asp Pro Gly Gln
   165 170 175
   Pro Ala Thr Ser Thr Ala Arg Pro Asn Arg Phe Gly Lys Lys Thr Met
   180 185 190
   Leu Cys Val Trp Trp Asp Gln Ser Gly Val Ile Tyr Tyr Glu Leu Leu
   195 200 205
   Lys Arg Gly Glu Thr Val Asn Thr Ala Arg Tyr Gln Gln Gln Leu Ile
   210 215 220
   Asn Leu Asn Arg Ala Leu Gln Arg Lys Arg Pro Glu Tyr Gln Lys Arg
   225 230 235 240
   Gln His Arg Val Ile Phe Leu His Asp Asn Ala Pro Ser His Thr Ala
   245 250 255
   Arg Ala Val Arg Asp Thr Leu Glu Thr Leu Asn Trp Glu Val Leu Pro
   260 265 270
   His Ala Ala Tyr Ser Pro Asp Leu Ala Pro Ser Asp Tyr His Leu Phe
   275 280 285
   Ala Ser Met Gly His Ala Leu Ala Glu Gln Arg Phe Asp Ser Tyr Glu
   290 295 300
   Ser Val Lys Lys Trp Leu Asp Glu Trp Phe Ala Ala Lys Asp Asp Glu
   305 310 315 320
   Phe Tyr Trp Arg Gly Ile His Lys Leu Pro Glu Arg Trp Glu Lys Cys
   325 330 335
   Val Ala Ser Asp Gly Lys Tyr Leu Glu
   340 345
<210> 3
   <211> 36
<212> DNA
   <213> Séquence artificielle
<220>
   <223> Amorce directe F53Y
<400> 3
   ggtggtttca acgctacaaa agtggtgatt ttgacg 36
<210> 4
   <211> 36
   <212> DNA
   <213> Séquence artificielle
<220>
   <223> Amorce inverse F53Y
<400> 4
   cgtcaaaatc accacttttg tagcgttgaa accacc 36
<210> 5
   <211> 28
   <212> DNA
   <213> Séquence artificielle
<220>
   <223> Amorce directe Q91R
<400> 5
   gctcaaacgc aaaaacgact cgcagagc 28
<210> 6
   <211> 28
   <212> DNA
   <213> Séquence artificielle
<220>
   <223> Amorce inverse Q91R
<400> 6
   gctctgcgag tcgtttttgc gtttgagc 28
<210> 7
   <211> 27
   <212> DNA
   <213> Séquence artificielle
<220>
   <223> Amorce directe L92A
<400> 7
   cgcaaaaaca agccgcagag cagttgg 27
<210> 8
   <211> 27
   <212> DNA
   <213> Séquence artificielle
<220>
   <223> Amorce inverse L92A
<400> 8
   ccaactgctc tgcggcttgt ttttgcg 27
<210> 9
   <211> 27
   <212> DNA
   <213> Séquence artificielle
<220>
   <223> Amorce directe L92R
<400> 9
   cgcaaaaaca acgcgcagag cagttgg 27
<210> 10
   <211> 27
   <212> DNA
   <213> Séquence artificielle
<220>
   <223> Amorce inverse L92R
<400> 10
   ccaactgctc tgcgcgttgt ttttgcg 27
<210> 11
   <211> 31
   <212> DNA
   <213> Séquence artificielle
<220>
   <223> Amorce directe Q100N
<400> 11
   gcagttggaa gtaagtaacc aagcagtttc c 31
<210> 12
   <211> 31
   <212> DNA
   <213> Séquence artificielle
<220>
   <223> Amorce inverse Q100N
<400> 12
   ggaaactgct tggttactta cttccaactg c 31
<210> 13
   <211> 31
   <212> DNA
   <213> Séquence artificielle
<220>
   <223> Amorce directe Q100R
<400> 13
   gcagttggaa gtaagtegac aagcagtttc c 31
<210> 14
   <211> 31
   <212> DNA
   <213> Séquence artificielle
<220>
   <223> Amorce inverse Q100R
<400> 14
   ggaaactgct tgtcgactta cttccaactg c 31
<210> 15
   <211> 31
   <212> DNA
   <213> Séquence artificielle
<220>
   <223> Amorce directe Q100E
<400> 15
   gcagttggaa gtaagtgaac aagcagtttc c 31
<210> 16
   <211> 31
   <212> DNA
   <213> Séquence artificielle
<220>
   <223> Amorce inverse Q100E
<400> 16
   ggaaactgct tgttcactta cttccaactg c 31
<210> 17
   <211> 44
   <212> DNA
   <213> Séquence artificielle
<220>
   <223> Amorce directe S104P
<400> 17
   ggaagtaagt caacaagcag ttcccaatcg cttgcgagag atgg 44
<210> 18
   <211> 44
   <212> DNA
   <213> Séquence artificielle
<220>
   <223> Amorce inverse S104P
<400> 18
   ccatctctcg caagcgattg ggaactgctt gttgacttac ttcc 44
<210> 19
   <211> 25
   <212> DNA
   <213> Séquence artificielle
<220>
   <223> Amorce directe N105A
<400> 19
   caagcagttt ccgcacgctt gcgag 25
<210> 20
   <211> 25
   <212> DNA
   <213> Séquence artificielle
<220>
   <223> Amorce inverse N105A
<400> 20
   ctcgcaagcg tgcggaaact gcttg 25
<210> 21
   <211> 34
   <212> DNA
   <213> Séquence artificielle
<220>
   <223> Amorce directe E137K
<400> 21
   ggcgcaaaaa cacatgcaaa attttgcttt cacg 34
<210> 22
   <211> 34
   <212> DNA
   <213> Séquence artificielle
<220>
   <223> Amorce inverse E137K
<400> 22
   cgtgaaagca aaattttgca tgtgtttttg cgcc 34
<210> 23
   <211> 27
   <212> DNA
   <213> Séquence artificielle
<220>
   <223> Amorce directe T216A
<400> 23
   gcgaaacggt gaatgcqgca cgctacc 27
<210> 24
   <211> 27
   <212> DNA
   <213> Séquence artificielle
<220>
   <223> Amorce inverse T216A
<400> 24
   ggtagcgtgc cgcattcacc gtttcgc 27
<210> 25
   <211> 43
   <212> DNA
   <213> Séquence artificielle
<220>
   <223> Amorce directe Y237C
<400> 25
   gcttcagaga aaacgaccgg aatgtcaaaa aagacaacac agg 43
<210> 26
   <211> 43
   <212> DNA
   <213> Séquence artificielle
<220>
   <223> Amorce inverse Y237C
<400> 26
   cctgtgttgt cttttttgac attccggtcg ttttctctga agc 43
<210> 27
   <211> 33
   <212> DNA
   <213> Séquence artificielle
<220>
   <223> Amorce directe W268F
<400> 27
   cgttggaaac actcaatttc gaagtgcttc cgc 33
<210> 28
   <211> 33
   <212> DNA
   <213> Séquence artificielle
<220>
   <223> Amorce inverse W268F
<400> 28
   gcggaagcac ttcgaaattg agtgtttcca acg 33
<210> 29
   <211> 33
   <212> DNA
   <213> Séquence artificielle
<220>
   <223> Amorce directe W268Y
<400> 29
   cgttggaaac actcaattac gaagtgcttc cgc 33
<210> 30
   <211> 33
   <212> DNA
   <213> Séquence artificielle
<220>
   <223> Amorce inverse W268Y
<400> 30 gcggaagcac ttcgtaattg agtgtttcca acg 33
<210> 31
   <211> 27
   <212> DNA
   <213> Séquence artificielle
<220>
   <223> Amorce directe W268A
<400> 31
   cgttggaaac actcaatgcg gaagtgc 27
<210> 32
   <211> 27
   <212> DNA
   <213> Séquence artificielle
<220>
   <223> Amorce inverse W268A
<400> 32
   gcacttccgc attgagtgtt tccaacg 27
<210> 33
   <211> 24
   <212> DNA <213> Séquence artificielle
<220>
   <223> Amorce PCR MTP up
<400> 33
   tacgtaatgt cgagtttcgt gccg 24
<210> 34
   <211> 24
   <212> DNA
   <213> Séquence artificielle
<220>
   <223> Amorce PCR 3'HindIII
<400> 34
   cccaagctta ttcaaagtat ttgc 24
<210> 35
   <211> 25
   <212> DNA
   <213> Séquence artificielle
<220> <223> Amorce R pour la méthode Selex
<400> 35
   caggtcagtt cagcggatcc tgtcg 25
<210> 36
   <211> 25
   <212> DNA
   <213> Séquence artificielle
<220>
   <223> Amorce F pour la méthode Selex
<400> 36
   gaggcgaatt cagtgcaact gcagc 25
<210> 37
   <211> 28
   <212> DNA
   <213> drosophila mauritiana
<220>
   <223> Séquence ITR3'
<400> 37
   tcaggtgtac aagtatgaaa tgtcgttt 28
<210> 38
   <211> 28
   <212> DNA
   <213> Séquence artificielle
<220>
   <223> Séquence selex46
<400> 38
   tcaggtgtac aagtatgaga tgtcgttt 28
<210> 39
   <211> 28
   <212> DNA
   <213> Séquence artificielle
<220>
   <223> Séquence selex40
<400> 39
   tcaggtgtac aagtatgtaa tgtcgtta 28
<210> 40
   <211> 28
   <212> DNA
   <213> Séquence artificielle
<220>
   <223> Séquence selex49
<400> 40
   gcgggtgtga aaatatgaaa tattcact 28
<210> 41
   <211> 28
   <212> DNA
   <213> Séquence artificielle
<220>
   <223> Séquence selex1
<400> 41
   gaagagttat aactcaagaa aactgaat 28
<210> 42
   <211> 25
   <212> DNA
   <213> Séquence artificielle
<220>
   <223> Séquence selex69
<400> 42
   cgattacaat tatgaaaact ttcct 25
<210> 43
   <211> 22
   <212> DNA
   <213> Séquence artificielle
<220>
   <223> Séquence selex6
<400> 43
   ggagaaatgg tagaatagtt ta 22
<210> 44
   <211> 19
   <212> DNA
   <213> Séquence artificielle
<220>
   <223> Séquence selex9
<400> 44
   atagtgttta aagcttact 19
<210> 45
   <211> 34
   <212> DNA
   <213> Séquence artificielle
<220>
   <223> Séquence selex60
<400> 45
   gtcagtgatg tcaccaacgt atggaatatc gtat 34